# EUROPEAN PATENT SPECIFICATION

(11) **EP 2 922 871 B1**
(45) Date of publication and mention of the grant of the patent: **19.06.2019**
(21) Application number: 13857297.9
(22) Date of filing: 20.11.2013
(51) Int. Cl.: C07K 14/745, C12N 15/12, C12N 5/10, A01K 67/027

(54) **METHODS AND COMPOSITIONS FOR MODIFIED FACTOR IX PROTEINS**
VERFAHREN UND ZUSAMMENSETZUNGEN FÜR MODIFIZIERTE FAKTOR-IX-PROTEINE
PROCÉDÉS ET COMPOSITIONS POUR PROTÉINES FACTEUR IX

(30) Priority: 20.11.2012 US 201261728469 P; 18.09.2013 US 201361879394 P
(43) Date of publication of application: 30.09.2015
(73) Proprietor: The University of North Carolina at Chapel Hill, Chapel Hill, NC 27599-4105 (US)
(72) Inventor: STAFFORD, Darrel W., Carrboro, North Carolina 27510 (US); FENG, Dengmin, Guangzhou 510530 (CN)
(74) Representative: HGF Limited
(86) International application number: PCT/US2013/071009
(87) International publication number: WO 2014/081831

(56) References cited:
- WO-A1-2012/061654
- WO-A2-2008/127654
- WO-A2-2009/051717
- US-A1- 2008 102 115
- US-A1- 2012 164 130
- US-B2- 7 700 734
- T. GUI ET AL: "Circulating and binding characteristics of wild-type factor IX and certain Gla domain mutants in vivo", BLOOD, vol. 100, no. 1, 17 June 2002 (2002-06-17) , pages 153-158, XP055273405, US ISSN: 0006-4971, DOI: 10.1182/blood.V100.1.153
- Cheung ET AL: "Identification of the endothelial cell binding site for factor IX WING-FAI Communicated by", Medical Sciences, 1 October 1996 (1996-10-01), pages 11068-11073, XP055273376, Retrieved from the Internet: URL:http://www.pnas.org/content/93/20/1106 8.full.pdf
- DATABASE GENBANK [Online] 24 October 2012 XP055256802 Retrieved from NCBI Database accession no. NP_000124.1
- CHEUNG, WING-FAI ET AL.: 'The binding of human factor IX to endothelial cells is mediated by residues 3-11' THE JOURNAL OF BIOLOGICAL CHEMISTRY vol. 267, no. 29, 1992, pages 20529 - 20531, XP055253322
- CHANG, JINLI ET AL.: 'Changing residue 338 in human factor IX from arginine to alanine causes an increase in catalytic activity' THE JOURNAL OF BIOLOGICAL CHEMISTRY vol. 273, no. 20, 1998, pages 12089 - 12094, XP002199691

## Description

### STATEMENT OF GOVERNMENT SUPPORT

This invention was made with government support under Grant No. HL006350 awarded by the National Institutes of Health. The government has certain rights in the invention.

### STATEMENT REGARDING ELECTRONIC FILING OF A SEQUENCE LISTING

A Sequence Listing in ASCII text format, submitted under 37 C.F.R. § 1.821, entitled 5470-643WO_ST25.txt, 26,453 bytes in size, generated on November 18, 2013 and filed via EFS-Web, is provided in lieu of a paper copy.

### BACKGROUND OF THE INVENTION

### Field of the Invention

The invention pertains to Factor IX proteins containing modifications in the amino acid sequence of the Factor IX protein, as well as nucleic acid constructs encoding the Factor IX proteins.

### Background of the invention

Factor IX is commercially available as both a plasma-derived product (Mononine®) and a recombinant protein (Benefix®). Mononine® has the disadvantage that there is a potential to transmit disease through contamination with bacteria and viruses (such as HIV, hepatitis) which are carried through the purification procedure. The use of recombinant protein (e.g., Benefix®) avoids these problems. However, the pharmacokinetic properties of recombinant Factor IX (rFactor IX, e.g., Benefix®) do not compare well with the properties of human plasma-derived Factor IX (pdFactor IX, e.g., Mononine®) after intravenous (i.v.) bolus infusion in laboratory animal model systems and in humans. Due to the less favorable pharmacokinetic properties of rFactor IX, generally 20-30% higher doses of rFactor IX are required to achieve the same procoagulant activity level as pdFactor IX (White et al. (April 1998) Seminars in Hematology vol. 35, no. 2 Suppl. 2: 33-38; Roth et al. (December 15, 2001) Blood vol. 98 (13): 3600-3606).

WO 2008/127655 relates to methods and compositions for treating blood clotting factor disorders and/or reducing bleeding-associated joint damage by treatments delivered to the joint in a subject. The present invention provides Factor IX (FIX) proteins having additional glycosylation sites and amino acid sequence modifications. The Factor IX proteins of this invention have higher specific activity and a longer useful clotting function relative to wild type or non-modified Factor IX protein.

### SUMMARY OF THE INVENTION

The present invention is further defined as set forth in the claims.

The present invention provides a FIX protein comprising the amino acid sequence (SEQ ID NO:3): wherein Xaa is leucine.

In a further embodiment, the present invention provides an isolated nucleic acid molecule encoding the isolated FIX protein comprising the amino acid set forth in SEQ ID NO:3, wherein Xaa is leucine. The nucleic acid molecule may comprise the nucleotide sequence (FIX 24-K5R codon optimized sequence with propeptide sequence and a R338L substitution) (SEQ ID NO:5): wherein NNN is any three nucleotide codon encoding leucine.

The present invention further provides a Factor IX protein, the nucleic acid molecule, the vector and/or the cell of this invention for use in treating a bleeding disorder.

Described herein for reference is an isolated Factor IX (FIX) protein comprising a K5R substitution (Lys to Arg substitution at position 5) in the amino acid sequence of SEQ ID NO:1:

The present invention provides an isolated Factor IX (FIX) protein comprising at least three additional glycosylation sites relative to wild type human FIX, a K5R substitution and a R338X substitution of the FIX amino acid sequence of SEQ ID NO:1, wherein X is leucine.
SEQ ID NO:1:

Described herein is a FIX protein comprising the amino acid sequence (SEQ ID NO:2): wherein X is any amino acid except R (arginine).

Described herein is an isolated nucleic acid molecule comprising the nucleotide sequence (FIX 24-K5R codon optimized sequence with propeptide sequence) (SEQ ID NO:4):

Additionally provided is a method of increasing the bioavailablity of a Factor IX protein in a subject, comprising administering to the subject an effective amount of the Factor IX protein, the nucleic acid molecule and/or the cell as described herein.

### BRIEF DESCRIPTION OF THE DRAWINGS

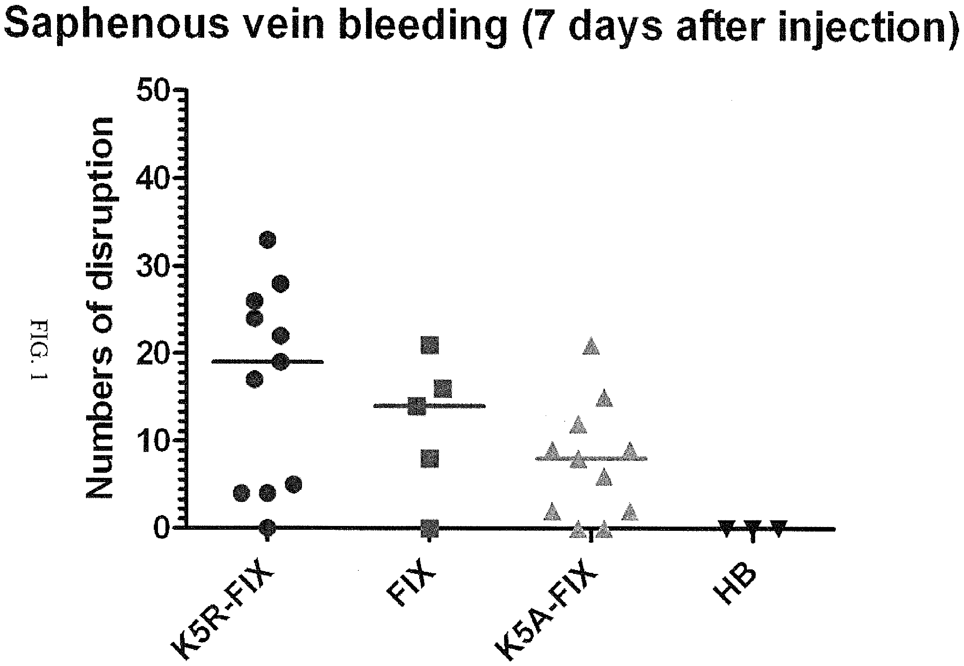

**Figure 1** shows the ability of wild type FIX (WTFIX) and it K5A (FIXK5A) and K5R (FIXK5R) variants to protect hemophilia B mice from bleeding 7 days after injection. *Factor IX* gene-ablated C57BL/6 mice were subjected to saphenous vein incision 7 days after injection of FIX or one of its variants. The number of times that bleeding stopped spontaneously was determined up to a limit of 30 minutes, as described herein. The median NOD (numbers of disruption) for K5RFIX was 19 and for K5AFIX was 8 (P < 0.05, unpaired t-test). WTFIX falls in between with 14 disruptions. Each point represents a single mouse.

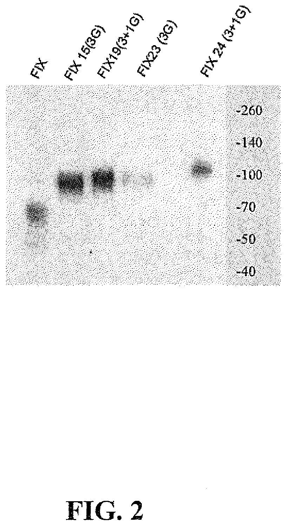

**Figure 2** shows the size of FIX variants with different glycosylation modifications. Lane 1. wt FIX; Lane 2. FIX 15, which has three extra glycosylation sites in the activation peptide; Lane 3. FIX 19. In addition to the same three extra glycosylation sites in the activation peptide as FIX15, FIX 19 has one more glycosylation site in the catalytic region; Lane 4. FIX 23, which also has three extra glycosylation sites in the activation peptide. Compared with FIX15, more amino acids were introduced between each site; Lane 6. FIX 24, in addition to the three extra sites in FIX 23, FIX 24 has one more glycosylation site in the catalytic region.

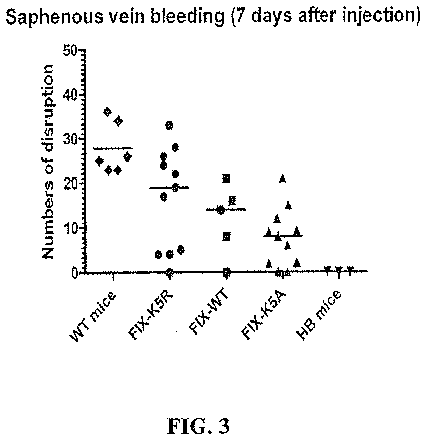

**Figure 3****.** Hemophilia B mice received a bolus injection (0.9 mg/kg) of: FIXK5R, which binds tighter than wild-type to collagen IV; FIXWT; and FIXK5A, which binds weaker than wild-type to collagen IV. Seven days after injection the ability of these molecules to promote haemostasis in a saphenous vein bleeding test was compared to wild type and hemophilia B mice. The P-values are from a one-sided Mann-Whitney model. The P value for FIX_{K5R} vs. FIX_{K5A} was 0.003.

Further aspects, features and advantages of this invention will become apparent from the detailed description of the embodiments which follow.

### DETAILED DESCRIPTION OF THE INVENTION

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention.

### Definitions

As used herein, "a," "an" or "the" can mean one or more than one. For example, "a" cell can mean a single cell or a multiplicity of cells.

Also as used herein, "and/or" refers to and encompasses any and all possible combinations of one or more of the associated listed items, as well as the lack of combinations when interpreted in the alternative ("or").

The term "about," as used herein when referring to a measurable value such as an amount (e.g., an amount of methylation) and the like, is meant to include variations of ±20%, ±10%, ±5%, ±1%, ±0.5%, or even ±0.1% of the specified amount.

As used herein, the transitional phrase "consisting essentially of" means that the scope of a claim is to be interpreted to encompass the specified materials or steps recited in the claim, "and those that do not materially affect the basic and novel characteristic(s)" of the claimed invention. *See, In re Herz,* 537 F.2d 549, 551-52, 190 U.S.P.Q. 461, 463 (CCPA 1976) (emphasis in the original); *see also* MPEP § 2111.03. Thus, the term "consisting essentially of" when used in a claim of this invention is not intended to be interpreted to be equivalent to "comprising."

The term "pharmacokinetic properties" has its usual and customary meaning and refers to the absorption, distribution, metabolism and excretion of the Factor IX protein.

The usual and customary meaning of "bioavailability" is the fraction or amount of an administered dose of a biologically active drug that reaches the systemic circulation. Described herein, the term "bioavailability" includes the usual and customary meaning but, in addition, is taken to have a broader meaning to include the extent to which the Factor IX protein is bioactive. In the case of Factor IX, for example, one measurement of "bioavailability" is the procoagulant activity of Factor IX protein obtained in the circulation post-infusion.

"Posttranslational modification" has its usual and customary meaning and includes but is not limited to removal of leader sequence, γ-carboxylation of glutamic acid residues, β-hydroxylation of aspartic acid residues, N-linked glycosylation of asparagine residues, O-linked glycosylation of serine and/or threonine residues, sulfation of tyrosine residues, phosphorylation of serine residues and any combination thereof.

As used herein, "biological activity" is determined with reference to a standard derived from human plasma. For Factor IX, the standard is MONONINE® (ZLB Behring). The biological activity of the standard is taken to be 100%.

The term "Factor IX protein" or "FIX protein" as used herein includes wild type Factor IX protein as well as naturally occurring or man-made proteins (e.g., the T/A dimorphism in the activation peptide of human FIX at position 148 (numbering based on the mature human FIX amino acid sequence of SEQ ID NO:1, which shows a T at position 148) as described in Graham et al. ("The Malmo polymorphism of coagulation factor IX, an immunologic polymorphism due to dimorphism of residue 148 that is in linkage disequilibrium with two other FIX polymorphisms" Am. J. Hum. Genet. 42:573-580 (1988)) Described herein is a FIX protein which includes a FIX protein having the amino acid sequence of SEQ ID NO:1, wherein the amino acid at position 148 can be a T or an A and a subject can be either heterozygous or homozygous for either T or A at this site. A FIX protein described herein can further include mutated forms of FIX as are known in the literature (see, e.g., Chang et al. "Changing residue 338 in human factor IX from arginine to alanine causes an increase in catalytic activity" J. Biol. Chem. 273:12089-94 (1998); Cheung et al. "Identification of the endothelial cell binding site for factor IX" PNAS USA 93:11068-73 (1996); Horst, Molecular Pathology, page 361 (458 pages) CRC Press, 1991,). A FIX protein as described herein further includes any other naturally occurring human FIX protein or man made human FIX protein now known or later identified, and derivatives and active fragments/active domains thereof, as are known in the art. A Factor IX protein of this invention further includes the pharmacologically active form of FIX, which is the molecule from which the activation peptide has been cleaved out of the protein by the action of proteases (or by engineering it out of the protein by removing it at the nucleic acid level), resulting in two non-contiguous polypeptide chains for FIX (light chain and heavy chain) folded as the functional FIX clotting factor. Specifically, Factor IX proteins having a modification to increase the degree of glycosylation are specifically included in the broad term.

The term "half life" is a broad term which includes the usual and customary meaning as well as the usual and customary meaning found in the scientific literature for Factor IX. Specifically included in this definition is a measurement of a parameter associated with Factor IX which defines the time post-infusion for a decrease from an initial value measured at infusion to half the initial value. In some embodiments, the half life of FIX can be measured in blood and/or blood components using an antibody to Factor IX in a variety of immunoassays, as are well known in the art and as described herein. Alternatively, half life may be measured as a decrease in Factor IX activity using functional assays including standard clotting assays, as are well known in the art and as described herein.

The term "recovery" as used herein includes the amount of FIX, as measured by any acceptable method including but not limited to FIX antigen levels or FIX protease or clotting activity levels, detected in the circulation of a recipient animal or human subject at the earliest practical time of removing a biological sample (e.g., a blood or blood product sample) for the purpose of measuring the level of FIX following its infusion, injection, delivery or administration otherwise. With current methodologies, the earliest biological sampling time for measuring FIX recovery typically falls within the first 15 minutes post infusion, injection, or delivery/administration otherwise of the FIX, but it is reasonable to expect quicker sampling times as scientific and/or clinical technologies improve. In essence, the recovery value for FIX is meant here to represent the maximum fraction of infused, injected or otherwise delivered/administered FIX that can be measured in the circulation of the recipient at the earliest possible time point following infusion, injection, or other delivery to a recipient animal or patient.

The term "glycosylation site(s)" is a broad term that has its usual and customary meaning. In the context of the present application the term applies to both sites that potentially could accept a carbohydrate moiety, as well as sites within the protein, specifically FIX, on which a carbohydrate moiety has actually been attached and includes any amino acid sequence that could act as an acceptor for oligosaccharide and/or carbohydrate.

The term "isolated" can refer to a nucleic acid or polypeptide that is substantially free of cellular material, viral material, and/or culture medium (when produced by recombinant DNA techniques), or chemical precursors or other chemicals (when chemically synthesized). Moreover, an "isolated fragment" is a fragment of a nucleic acid or polypeptide that is not naturally occurring as a fragment and would not be found in the natural state.

An "isolated cell" refers to a cell that is separated from other cells and/or tissue components with which it is normally associated in its natural state. For example, an isolated cell is a cell that is part of a cell culture. An isolated cell can also be a cell that is administered to or introduced into a subject, e.g., to impart a therapeutic or otherwise beneficial effect.

### Compositions of the invention

Described herein is an isolated Factor IX (FIX) protein comprising a K5R substitution in the amino acid sequence of SEQ ID NO:1:

In some cases, the Lys at position 5 of the amino acid sequence of SEQ ID NO:1 can be substituted with threonine, leucine or isoleucine, as nonlimiting examples. Any substitution of the Lys at position 5 that results in a Factor XI molecule that increases the affinity between Factor IX and type IV collagen is described herein.

In further cases of the FIX protein described herein,
the valine (Val) at position 10 in the amino acid sequence of SEQ ID NO:1 can be substituted with the following nonlimiting examples: leucine, isoleucine, methionine or phenylalanine, histidine or threonine.

The FIX protein described herein can be a FIX protein with a substitution at position 5 as described herein and/or a substitution at position 10 as described herein and/or a substitution of the phenylalanine (Phe) at position 9 of the amino acid sequence of SEQ ID NO:1 with any other amino acid.

The FIX protein described herein can be a FIX protein with a substitution at position 5 as described herein and/or a substitution at position 10 as described herein and/or a substitution of the phenylalanine (Phe) at position 9 of the amino acid sequence of SEQ ID NO:1 with any other amino acid and/or a substitution of the glutamine (Gln) at position 11 with the following nonlimiting examples: asparagine, lysine or arginine.

The substitutions as described herein at positions 5, 9, 10 and 11 of the amino acid sequence of SEQ ID NO:1 can be present singly or in any combination.

The Factor IX protein with the substitutions as described herein at positions 5, 9, 10 and 11, singly or in combination, can further comprise one or more (e.g., 1, 2, 3, 4, 5, 6, 7, 8, 9, 10, 11, 12, 13, 14, 15, 16, 17, 18, 19, 20, etc.) additional glycosylation sites relative to wild type human FIX.

The present invention provides an isolated Factor IX (FIX) protein comprising at least three additional glycosylation sites relative to wild type human FIX, a K5R substitution and a R338X substitution of the FIX amino acid sequence of SEQ ID NO:1, wherein X is leucine.
SEQ ID NO:1:

Described herein is an isolated Factor IX (FIX) protein comprising at least three additional glycosylation sites relative to wild type human FIX, a K51R substitution and/or a R384X substitution of the FIX amino acid sequence of SEQ ID NO:6, wherein X is an amino acid other than arginine.

Described herein is a FIX protein comprising the amino acid sequence (SEQ ID NO:7) of: wherein X is any amino acid except R (arginine).

Also described herein is a FIX protein comprising the amino acid sequence (SEQ ID NO:2): wherein X is any amino acid except R (arginine).

Also provided herein is a FIX protein comprising the amino acid sequence (SEQ ID NO:3): wherein Xaa is leucine. In some cases described herein, Xaa can be any amino acid except arginine, including for example, any such amino acid listed herein in **Table 1.**

Further described herein is an isolated nucleic acid molecule comprising the nucleotide sequence (FIX 24-K5R codon optimized sequence with propeptide sequence) (SEQ ID NO:4):

In further embodiments, the present invention provides an isolated nucleic acid molecule comprising the nucleotide sequence (FIX 24-K5R codon optimized sequence with propeptide sequence and a R338L substitution) (SEQ ID NO:5): wherein NNN is any three nucleotide codon encoding leucine.

The present invention also provides a Factor IX protein, the nucleic acid molecule, the vector and/or the cell of any this invention for use in treating a bleeding disorder.

Some embodiments of the invention are directed to Factor IX proteins having one or more (e.g., 1, 2, 3, 4, 5, 6, 7, 8, 9, 10, 11, 12, 13, 14, 15, etc.) additional glycosylation sites. By "additional" or "new" glycosylation sites is meant that the number of glycosylation sites in the FIX protein is greater than the number of glycosylation sites normally present in a "wild type" form of Factor IX. A Factor IX protein of this invention can include plasma derived FIX as well as recombinant forms of FIX. Generally, embodiments of the invention are directed to increasing the number of glycosylation sites in a FIX molecule of this invention. However, it is to be understood that a Factor IX protein of this invention that can be modified to increase the number of glycosylation sites and/or to increase the number of sugar chains is not limited to a particular "wild type" FIX amino acid sequence because naturally occurring or man-made FIX proteins can also be modified according to the methods of this invention to increase the number of glycosylation sites and/or to increase the number of sugar chains.

The present invention is further directed to FIX proteins containing one or more (e.g., 2, 3, 4, 5, 6, 7, 8, 9, 10, 11, 12, 13, 14, 15, etc.) additional sugar chains. Such additional sugar side chains can be present at one or more of the additional glycosylation sites introduced into the FIX proteins of this invention by the methods described herein. Alternatively, the additional sugar side chains can be present at sites on the FIX protein as a result of chemical and/or enzymatic methods to introduce such sugar chains to the FIX molecule, as are well known in the art. By "additional" or "new" sugar chains is meant that the number of sugar chains in the FIX protein is greater than the number of sugar chains normally present in a "wild type" form of Factor IX. In various embodiments, about 1 to about 50 additional sugar side chains (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) can be added.

In some embodiments, at least one additional glycosylation site is in the activation peptide of Factor IX (e.g., the human FIX activation peptide having the amino acid sequence of SEQ ID NO:1). The FIX protein described herein may have an insertion of a peptide segment that introduces one or more glycosylation sites between position N157 and N167 of the Factor IX amino acid sequence of SEQ ID NO:1.

Insertion(s) can be introduced into a FIX protein described herein to increase the number of glycosylation sites and such insertion(s) can include from about one to about 100 amino acid residues, including any number of amino acid residues from one to 100 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100).

In some cases, the insertion can include all or at least part (e.g., at least 3, 4, 5, 6, 7, 8, 9, 10, 11, 12, 13, 14, 15 or more amino acid residues) of a Factor IX activation peptide from a non-human species, such as mouse. This inserted peptide sequence can be further modified to introduce additional glycosylation sites according to the teachings herein.

The glycosylation site(s) may be N-linked glycosylation site(s). In some embodiments, the added glycosylation site(s) include N-linked glycosylation site(s) and the consensus sequence is NXT/S, with the proviso that X is not proline.

In some embodiments about one to about 15 glycosylation site(s) can be added to the FIX amino acid sequence. In various embodiments, about 1 to about 50 glycosylation site(s) (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) can be added. Described herein are FIX proteins in which a glycosylation site has been created by insertion, deletion or substitution of specific amino acids. In particular cases, the insertion, deletion and/or substitution is in the region of the activation peptide. The amino acid sequence of the human FIX activation peptide is provided herein as: Ala Glu Thr Val Phe Pro Asp Val Asp Tyr Val Asn Ser Thr Glu Ala Glu Thr Ile Leu Asp Asn Ile Thr Gln Ser Thr Gln Ser Phe Asn Asp Phe Thr Arg (SEQ ID NO:11).

It is contemplated that the additional glycosylation sites introduced into a FIX amino acid sequence can be introduced anywhere throughout the amino acid sequence of the FIX protein. Thus, in some cases, the additional glycosylation site or sites are introduced in the activation peptide (amino acids 146-180 of the mature human FIX amino acid sequence of SEQ ID NO:1). In some embodiments, the additional glycosylation site or sites are introduced outside the activation peptide (e.g., before and/or after the activation peptide). In some cases, the additional glycosylation site or sites are introduced both inside the activation peptide and outside the activation peptide. Thus, based on the numbering of the 415 amino acids of the amino acid sequence of the mature human FIX protein as shown in SEQ ID NO:1, a glycosylation attachment site can be introduced by inserting additional amino acid residues between or at any of amino acids 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122,123, 124, 125, 126, 127, 128, 129, 130, 131, 132, 133, 134, 135, 136, 137, 138, 139, 140,141, 142, 143, 144, 145, 146, 147, 148, 149, 150, 151, 152, 153, 154, 155,156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415 and any combination thereof. As used herein, a "glycosylation attachment site" or "glycosylation site" can mean a sugar attachment consensus sequence (i.e., a series of amino acids that act as a consensus sequence for attaching a sugar (mono-, oligo-, or poly-saccharide) to an amino acid sequence or it can mean the actual amino acid residue to which the sugar moiety is covalently linked. The sugar moiety can be a monosaccharide (simple sugar molecule), an oligosaccharide, or a polysaccharide.

In particular instances, additional amino acids can be inserted between and/or substituted into any of the amino acid residues that make up the activation peptide, such as between any of amino acids 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182 and any combination thereof. Furthermore, the same insert of this instance can be introduced multiple times at the same and/or at different locations in the amino acid sequence of the FIX protein, including within the activation peptide. Also, different inserts and/or the same inserts can be introduced one or more times at the same and/or at different locations between amino acid residues throughout the amino acid sequence of the FIX protein, including within the activation peptide. In one nonlimiting example, a glycosylation site can be added at amino acids 103, 151 and 228.

It is well known in the art that some proteins can support a large number of sugar side chains and the distance between N-linked glycosylation sites can be as few as three, four, five or six amino acids (see, e.g., Lundin et al. "Membrane topology of the Drosophila OR83b odorant receptor" FEBS Letters 581:5601-5604 (2007); Apweiler et al. "On the frequency of protein glycosylation, as deduced from analysis of the SWISS-PROT database" Biochimica et Biophysica Acta 1473:4-8 (19991),).

Furthermore, the FIX protein defined herein can be modified by mutation (e.g., substitution, addition and/or deletion of amino acids) to introduce N-linked glycosylation sites. For example, amino acid residues on the surface of the functional FIX protein can be identified according to molecular modeling methods standard in the art that would be suitable for modification (e.g., mutation) to introduce one or more glycosylation sites.

FIX proteins of this invention having additional glycosylation sites may be produced by recombinant methods such as site-directed mutagenesis using PCR. Alternatively, the Factor IX protein of this invention may be chemically synthesized to prepare a Factor IX protein with one or more (e.g., 1, 2, 3, 4, 5, 6, 7, 8, 9, 10, 11, 12, 13, 14, 15, etc.) additional glycosylation sites.

It is within the skill of one of ordinary skill in the art to modify any amino acid residue or residues in the mature FIX amino acid sequence according to methods well known in the art and as taught herein and to test any resulting FIX protein for activity, stability, recovery, half life, etc., according to well known methods and as described herein (see, e.g., Elliott et al. "Structural requirements for additional N-linked carbohydrate on recombinant human erythropoietin" J. Biol. Chem. 279:16854-62 (2004)).

Embodiments of the invention are directed to recombinant Factor IX proteins in which glycosylation sites have been added to improve the recovery and/or half-life and/or stability of Factor IX. The glycosylation sites may be N-linked glycosylation sites. In specific embodiments, at least one N-linked glycosylation site is added.

As noted herein, in some embodiments, at least one additional glycosylation site is introduced into the FIX amino acid sequence at a site that is outside of the activation peptide. In some embodiments, the at least one additional glycosylation site corresponds to a site that is glycosylated in the native form of a non-human homolog of Factor IX. A modification of the human FIX amino acid sequence to introduce a serine or threonine at amino acid 262 of the amino acid sequence of SEQ ID NO:1, which is the mature (i.e., secreted) form of human FIX, would introduce an additional N-linked glycosylation site in the human protein. In various instances, the non-human homolog is from dog, pig, cow, or mouse.

Additionally provided herein is a nucleic acid comprising, consisting essentially of and/or consisting of a nucleotide sequence encoding a FIX amino acid sequence of this invention. Such nucleic acids can be present in a vector, such as an expression cassette. Thus, further embodiments of the invention are directed to expression cassettes designed to express a nucleotide sequence encoding any of the Factor IX proteins of this invention. The nucleic acids and/or vectors of t his invention can be present in a cell. Thus, various embodiments of the invention are directed to recombinant host cells containing the vector (e.g., expression cassette). Such a cell can be isolated and/or present in a non-human transgenic animal. Therefore, certain embodiments of the invention are further directed to a non-human transgenic animal comprising a nucleic acid comprising a nucleotide sequence encoding any of the Factor IX proteins of the present invention.

A comparison of the amino acid sequence of the activation peptide of human, mouse, guinea pig and platypus FIX reveals that the mouse FIX amino acid sequence has an additional nine amino acids present in its activation peptide, the guinea pig FIX amino acid sequence has an additional ten amino acid residues present in its activation peptide and the platypus has an additional 14 amino acid residues present in its activation peptide. These extra amino acids are between the two naturally occurring glycosylation sites (N 157 and N 167) in human Factor IX.

The human and mouse FIX have essentially identical structures and the human enzyme can function in the mouse. As the human FIX functions without the additional nine amino acid segment found in the mouse, this region of the Factor IX molecule can tolerate modifications within its sequence, including insertions, substitutions and/or deletions, without substantial loss in structural, biochemical, or otherwise functional integrity of the molecule. The inserted nine amino acids in mouse are most likely surface residues (as supported by structural studies) and therefore accessible for modification by the glycosylation enzymes. In native human factor IX, the two N-linked glycosylation sites are 12 and 14 amino acids distant from the amino and carboxyl cleavage sites, respectively, of the activation peptide. Thus, in some cases described herein, additional amino acid residues can be added between N157 and N167 of the human Factor IX protein of SEQ ID NO:1 in order to add glycosylation sites to improve half life and/or bioavailability. In various cases, glycosylation sites are added by insertion, deletion and/or modification of the native sequence to include an attachment sequence for consensus sequences for N-linked glycosylation.

The human sequence for the activation peptide starts at residue 146 of the mature protein. The natural glycosylation sites are at N157 and N167 (SEQ ID NO:1). In some instances described herein, additional amino acid residues can be inserted between the two normal glycosylation sites (between N157 and N167 in the mature sequence) to provide additional glycosylation sites. In some cases described herein, about 3 to about 100 additional amino acid residues are added. In other cases described herein, about 5 to about 50 amino acid residues are added. In further cases described herein, about 5 to about 20 amino acid residues are added. In yet further cases described herein, about 7 to about 15 amino acid residues are added. Typically, the amino acid residues are chosen from the 20 biological amino acids with the proviso that proline is not used as "X" in the glycosylation site NXT/S, which is the consensus sequence for N-linked glycosylation. **Table 1** shows 20 common biological amino acids and their abbreviations.

N-glycosylation sites may be added. Consensus sequences for addition of glycosylation sites are known in the art and include the consensus sequence "NXT/S" for N-glycosylation where X is not proline.

In some cases described herein, endogenous N-linked attachment sequences from mouse, human and other mammalian Factor IX sequences are inserted into the activation peptide. These may be inserted individually or in combination. In certain cases described herein, the inserted segment includes a spacer region between glycosylation sites, which can be present individually, in tandem repeats, in multiples, etc. A spacer region of this invention can be from one to about 100 amino acids in length (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100). In some cases, for example, the spacer region can be from one to about 20 amino acids. In other cases the spacer region can be from one to about ten amino acids. In further cases, the spacer region can be from one to about five amino acid residues.

A spacer region described herein is included between the added carbohydrate attachment sites and/or between naturally occurring glycosylation sites and added glycosylation sites to reduce or eliminate steric hindrance and provide efficient recognition by the appropriate glycosyltransferase. A spacer region described herein can be comprised of any combination of amino acid residues provided that they are not cysteine or proline and provided that the amino acid sequence of the spacer does not have more than about 10% residues that are hydrophobic (e.g., tryptophan, tyrosine, phenylalanine and valine).

In some embodiments, NXT/S is incorporated into the inserted amino acid sequence to add one or more additional glycosylation sites. "X" may be any biological amino acid except that proline is disfavored. In some embodiments, at least one additional glycosylation site is added to the Factor IX protein. In other embodiments, two additional glycosylation sites are added. In further embodiments, three additional glycosylation sites are added. In yet further embodiments, four additional glycosylation sites are added. In further embodiments, five additional glycosylation sites are added. In some embodiments, six additional glycosylation sites are added. In other embodiments, more than six additional glycosylation sites are added.

In one case described herein, Ala at position 161 of the mature FIX amino acid sequence (SEQ ID NO: 1) is replaced with Asn to provide one additional glycosylation site. In a further case described herein, the sequence VFIQDNITD (SEQ ID NO:8) is inserted between residues 161 and 162 of the mature human FIX amino acid sequence of SEQ ID NO:1 to introduce an N-linked glycosylation site in the human FIX sequence. In yet a further case described herein, another new glycosylation site is added by replacing Asp with Asn in the VFIQDNITD insert. The inserted sequence would give VFIQDNITN (SEQ ID NO:9). The embodiments discussed above could be combined to provide one to four additional glycosylation sites in the human Factor IX protein.

In another embodiment, the following sequence is added, which provides five additional glycosylation sites. The glycosylation sites are shown in bold and underlined. AETVFPDVDYV**N**STE**N**ETIQD**N**ITD**N**ETILD**N**ITQSTQSFNDFTR (SEQ ID NO: 10)

In some embodiments, glycosylation sites are added at sites outside of the activation peptide. These additional sites can be selected, for example, by aligning the amino acid sequence of Factor IX from human with the Factor IX amino acid sequence from other species and determining the position of glycosylation sites in non-human species. The homologous or equivalent position in the human FIX amino acid sequence is then modified to provide a glycosylation site. This method may be used to identify both potential N-glycosylation and O-glycosylation sites.

The FIX proteins according to the invention are produced and characterized by methods well known in the art and as described herein. These methods include determination of clotting time (partial thromboplastin time (PPT) assay) and administration of the FIX protein to a test animal to determine recovery, half life, and bioavailability by an appropriate immunoassay and/or activity-assay, as are well known in the art.

The Factor IX protein, nucleic acid, vector and/or cell of this invention can be included in a pharmaceutical composition. Some embodiments are directed to a kit which includes the Factor IX protein of this invention.

The Factor IX protein of this invention can be for use in the treatment of a bleeding disorder in a subject (e.g., a human patient) in need thereof. Thus, the present invention also provides for the use of the Factor IX protein, the nucleic acid molecule, the vector and/or the cell of this invention for treating a bleeding disorder.

Also described herein is a method of increasing the bioavailablity of a Factor IX protein in a subject, comprising administering to the subject an effective amount of the Factor IX protein, the nucleic acid molecule and/or the cell described herein.

Bleeding disorders that can be treated according to this invention include a FIX deficiency, hemophilia B and Christmas disease. Such treatment protocols and dosing regimens for administering or delivering Factor IX to a subject in need thereof are well known in the art.

Many expression vectors can be used to create genetically engineered cells. Some expression vectors are designed to express large quantities of recombinant proteins after amplification of transfected cells under a variety of conditions that favor selected, high expressing cells. Some expression vectors are designed to express large quantities of recombinant proteins without the need for amplification under selection pressure. The present invention includes the production of genetically engineered cells according to methods standard in the art and is not dependent on the use of any specific expression vector or expression system.

To create a genetically engineered cell to produce large quantities of a Factor IX protein, cells are transfected with an expression vector that contains the cDNA encoding the protein. In some embodiments, the target protein is expressed with selected co-transfected enzymes that cause proper post-translational modification of the target protein to occur in a given cell system.

The cell may be selected from a variety of sources, but is otherwise a cell that may be transfected with an expression vector containing a nucleic acid, preferably a cDNA encoding a Factor IX protein.

The practice of the present invention employs, unless otherwise indicated, conventional techniques of molecular biology, microbiology, recombinant DNA, and immunology, which are within the skill of the art. Such techniques are explained fully in the literature. See, e.g., Sambrook, et al., Molecular Cloning; A Laboratory Manual, 2nd ed. (1989); DNA Cloning, Vols. I and II (D. N Glover, ed. 1985); Oligonucleotide Synthesis (M. J. Gait, ed. 1984); Nucleic Acid Hybridization (B. D. Hames & S. J. Higgins, eds. 1984); Transcription and Translation (B. D. Hames & S. J. Higgins, eds. 1984); Animal Cell Culture (R. I. Freshney, ed. 1986); Immobilized Cells and Enzymes (IRL Press, 1986); B. Perbal, A Practical Guide to Molecular Cloning (1984); the series, Methods in Enzymology (Academic Press, Inc.), particularly Vols. 154 and 155 (Wu and Grossman, and Wu, eds., respectively); Gene Transfer Vectors for Mammalian Cells (J. H. Miller and M. P. Calos, eds. 1987, Cold Spring Harbor Laboratory); Immunochemical Methods in Cell and Molecular Biology, Mayer and Walker, eds. (Academic Press, London, 1987); Scopes, Protein Purification: Principles and Practice, 2nd ed. 1987 (Springer-Verlag, N.Y.); and Handbook of Experimental Immunology Vols I-IV (D. M. Weir and C. C. Blackwell, eds 1986).

### Genetic Engineering Techniques

The production of cloned genes, recombinant DNA, vectors, transformed host cells, proteins and protein fragments by genetic engineering is well known. See, e.g., U.S. Pat. No. 4,761,371 to Bell et al. at Col. 6, line 3 to Col. 9, line 65; U.S. Pat. No. 4,877,729 to Clark et al. at Col. 4, line 38 to Col. 7, line 6; U.S. Pat. No. 4,912,038 to Schilling at Col. 3, line 26 to Col. 14, line 12; and U.S. Pat. No. 4,879,224 to Wallner at Col. 6, line 8 to Col. 8, line 59.

A vector is a replicable DNA construct. Vectors are used herein either to amplify nucleic acid encoding Factor IX protein and/or to express nucleic acid which encodes Factor IX protein. An expression vector is a replicable nucleic acid construct in which a nucleotide sequence encoding a Factor IX protein is operably linked to suitable control sequences capable of effecting the expression of the nucleotide sequence to produce a Factor IX protein in a suitable host. The need for such control sequences will vary depending upon the host selected and the transformation method chosen. Generally, control sequences include a transcriptional promoter, an optional operator sequence to control transcription, a sequence encoding suitable mRNA ribosomal binding sites, and sequences that control the termination of transcription and translation.

Vectors comprise plasmids, viruses (e.g., adenovirus, cytomegalovirus), phage, and integratable DNA fragments (i.e., fragments integratable into the host genome by recombination). The vector replicates and functions independently of the host genome, or may, in some instances, integrate into the genome itself. Expression vectors can contain a promoter and RNA binding sites that are operably linked to the gene to be expressed and are operable in the host organism.

DNA regions or nucleotide sequences are operably linked or operably associated when they are functionally related to each other. For example, a promoter is operably linked to a coding sequence if it controls the transcription of the sequence; or a ribosome binding site is operably linked to a coding sequence if it is positioned so as to permit translation of the sequence.

Transformed host cells are cells which have been transformed, transduced and/or transfected with Factor IX protein vector(s) constructed using recombinant DNA techniques.

Suitable host cells include prokaryote, yeast or higher eukaryotic cells such as mammalian cells and insect cells. Cells derived from multicellular organisms are a particularly suitable host for recombinant Factor IX protein synthesis, and mammalian cells are particularly preferred. Propagation of such cells in cell culture has become a routine procedure (Tissue Culture, Academic Press, Kruse and Patterson, editors (1973)). Examples of useful host cell lines are VERO and HeLa cells, Chinese hamster ovary (CHO) cell lines, and WI138, HEK 293, BHK, COS-7, CV, and MDCK cell lines. Expression vectors for such cells ordinarily include (if necessary) an origin of replication, a promoter located upstream from the nucleotide sequence encoding Factor IX protein to be expressed and operatively associated therewith, along with a ribosome binding site, an RNA splice site (if intron-containing genomic DNA is used), a polyadenylation site, and a transcriptional termination sequence. In a preferred embodiment, expression is carried out in Chinese Hamster Ovary (CHO) cells using the expression system of U.S. Patent No. 5,888,809.

The transcriptional and translational control sequences in expression vectors to be used in transforming vertebrate cells are often provided by viral sources. For example, commonly used promoters are derived from polyoma, Adenovirus 2, and Simian Virus 40 (SV40). See. e.g., U.S. Pat. No. 4,599,308.

An origin of replication may be provided either by construction of the vector to include an exogenous origin, such as may be derived from SV 40 or other viral (e.g., polyoma, adenovirus, VSV, or BPV) source, or may be provided by the host cell chromosomal replication mechanism. If the vector is integrated into the host cell chromosome, the latter is often sufficient.

Rather than using vectors which contain viral origins of replication, one can transform mammalian cells by the method of cotransformation with a selectable marker and the nucleic acid encoding the Factor IX protein. Examples of suitable selectable markers are dihydrofolate reductase (DHFR) or thymidine kinase. This method is further described in U.S. Pat. No. 4,399,216.

Other methods suitable for adaptation to the synthesis of Factor IX protein in recombinant vertebrate cell culture include those described in Gething et al. Nature 293:620 (1981); Mantei et al. Nature 281:40; and Levinson et al., EPO Application Nos. 117,060A and 117,058A.

Host cells such as insect cells (e.g., cultured *Spodoptera frugiperda* cells) and expression vectors such as the baculovirus expression vector (e.g., vectors derived from *Autographa californica* MNPV, *Trichoplusia ni* MNPV, *Rachiplusia ou* MNPV, or *Galleria ou* MNPV) may be employed in carrying out the present invention, as described in U.S. Pat. Nos. 4,745,051 and 4,879,236 to Smith et al. In general, a baculovirus expression vector comprises a baculovirus genome containing the nucleotide sequence to be expressed inserted into the polyhedrin gene at a position ranging from the polyhedrin transcriptional start signal to the ATG start site and under the transcriptional control of a baculovirus polyhedrin promoter.

Prokaryote host cells include gram negative or gram positive organisms, for example *Escherichia coli* (*E. coli*) or bacilli. Higher eukaryotic cells include established cell lines of mammalian origin as described below. Exemplary host cells are *E. coli* W3110 (ATCC 27,325), *E. coli* B, *E. coli* X1776 (ATCC 31,537) and *E. coli* 294 (ATCC 31,446). A broad variety of suitable prokaryotic and microbial vectors are available. *E. coli* is typically transformed using pBR322. Promoters most commonly used in recombinant microbial expression vectors include the betalactamase (penicillinase) and lactose promoter systems (Chang et al. Nature 275:615 (1978); and Goeddel et al. Nature 281:544 (1979)), a tryptophan (trp) promoter system (Goeddel et al. Nucleic Acids Res. 8:4057 (1980) and EPO App. Publ. No. 36,776) and the tac promoter (De Boer et al. Proc. Natl. Acad. Sci. USA 80:21 (1983)). The promoter and Shine-Dalgarno sequence (for prokaryotic host expression) are operably linked to the nucleic acid encoding the Factor IX protein, i.e., they are positioned so as to promote transcription of Factor IX messenger RNA from DNA.

Eukaryotic microbes such as yeast cultures may also be transformed with protein-encoding vectors (see, e.g., U.S. Pat. No. 4,745,057). *Saccharomyces cerevisiae* is the most commonly used among lower eukaryotic host microorganisms, although a number of other strains are commonly available. Yeast vectors may contain an origin of replication from the 2 micron yeast plasmid or an autonomously replicating sequence (ARS), a promoter, nucleic acid encoding Factor IX protein, sequences for polyadenylation and transcription termination, and a selection gene. An exemplary plasmid is YRp7, (Stinchcomb et al. Nature 282:39 (1979); Kingsman et al. Gene 7:141 (1979); Tschemper et al. Gene 10:157 (1980)). Suitable promoting sequences in yeast vectors include the promoters for metallothionein, 3-phosphoglycerate kinase (Hitzeman et al. J. Biol. Chem. 255:2073 (1980) or other glycolytic enzymes (Hess et al. J. Adv. Enzyme Reg. 7:149 (1968); and Holland et al. Biochemistry 17:4900 (1978)). Suitable vectors and promoters for use in yeast expression are further described in R. Hitzeman et al., EPO Publn. No. 73,657.

Cloned coding sequences described herein invention may encode FIX of any species of origin, including mouse, rat, dog, opossum, rabbit, cat, pig, horse, sheep, cow, guinea pig, opossum, platypus, and human, but preferably encode Factor IX protein of human origin. Nucleic acid encoding Factor IX that is hybridizable with nucleic acid encoding proteins disclosed herein is also encompassed. Hybridization of such sequences may be carried out under conditions of reduced stringency or even stringent conditions (e.g., stringent conditions as represented by a wash stringency of 0.3M NaCl, 0.03M sodium citrate, 0.1% SDS at 60°C or even 70°C) to nucleic acid encoding Factor IX protein disclosed herein in a standard *in situ* hybridization assay. See, e.g., Sambrook et al., Molecular Cloning, A Laboratory Manual (2d Ed. 1989) Cold Spring Harbor Laboratory).

The FIX proteins produced according to the invention may be expressed in non-human transgenic animals by known methods. See for example, U.S. Patent No. 6,344,596. In brief, transgenic animals may include but are not limited to farm animals (e.g., pigs, goats, sheep, cows, horses, rabbits and the like) rodents (such as mice, rats and guinea pigs), and domestic pets (for example, cats and dogs). Livestock animals such as pigs, sheep, goats and cows, are particularly preferred in some embodiments.

The non-human transgenic animal of this invention is produced by introducing into a single cell non-human embryo an appropriate polynucleotide that encodes a human Factor IX protein of this invention in a manner such that the polynucleotide is stably integrated into the DNA of germ line cells of the mature animal, and is inherited in normal Mendelian fashion. The non-human transgenic animal of this invention would have a phenotype of producing the FIX protein in body fluids and/or tissues. The FIX protein would be removed from these fluids and/or tissues and processed, for example for therapeutic use. (See, e.g., Clark et al. "Expression of human anti-hemophilic factor IX in the milk of transgenic sheep" Bio/Technology 7:487-492 (1989); Van Cott et al. "Haemophilic factors produced by transgenic livestock: abundance can enable alternative therapies worldwide" Haemophilia 10(4):70-77 (2004)).

DNA molecules can be introduced into non-human embryos by a variety of means including but not limited to microinjection, calcium phosphate mediated precipitation, liposome fusion, or retroviral infection of totipotent or pluripotent stem cells. The transformed cells can then be introduced into non-human embryos and incorporated therein to form non-human transgenic animals. Methods of making transgenic non-human animals are described, for example, in Transgenic Animal Generation and Use by L. M. Houdebine, Harwood Academic Press, 1997. Transgenic animals also can be generated using methods of nuclear transfer or cloning using embryonic or adult cell lines as described for example in Campbell et al., Nature 380:64-66 (1996) and Wilmut et al., Nature 385:810-813 (1997). Further a technique utilizing cytoplasmic injection of DNA can be used as described in U.S. Pat. No. 5,523,222.

Factor IX-producing non-human transgenic animals can be obtained by introducing a chimeric construct comprising Factor IX-encoding sequences. Methods for obtaining transgenic animals are well-known. See, for example, Hogan et al., MANIPULATING THE MOUSE EMBRYO, (Cold Spring Harbor Press 1986); Krimpenfort et al., Bio/Technology 9:88 (1991); Palmiter et al., Cell 41:343 (1985), Kraemer et al., GENETIC MANIPULATION OF THE EARLY MAMMALIAN EMBRYO, (Cold Spring Harbor Laboratory Press 1985); Hammer et al., Nature 315:680 (1985); Wagner et al., U.S. Pat. No. 5,175,385; Krimpenfort et al., U.S. Pat. No. 5,175,384, Janne et al., Ann. Med. 24:273 (1992), Brem et al., Chim. Oggi. 11:21 (1993), Clark et al., U.S. Pat. No. 5,476,995.

In some embodiments, cis-acting regulatory regions may be used that are "active" in mammary tissue in that the promoters are more active in mammary tissue than in other tissues under physiological conditions where milk is synthesized. Such promoters include but are not limited to the short and long whey acidic protein (WAP), short and long α, β and κ casein, α-lactalbumin and β-lactoglobulin ("BLG") promoters. Signal sequences can also be used in accordance with this invention that direct the secretion of expressed proteins into other body fluids, particularly blood and urine. Examples of such sequences include the signal peptides of secreted coagulation factors including signal peptides of Factor IX, protein C, and tissue-type plasminogen activator.

Among the useful sequences that regulate transcription, in addition to the promoters discussed above, are enhancers, splice signals, transcription termination signals, polyadenylation sites, buffering sequences, RNA processing sequences and other sequences which regulate the expression of transgenes.

Preferably, the expression system or construct includes a 3' untranslated region downstream of the nucleotide sequence encoding the desired recombinant protein. This region can increase expression of the transgene. Among the 3' untranslated regions useful in this regard are sequences that provide a poly A signal.

Suitable heterologous 3'-untranslated sequences can be derived, for example, from the SV40 small t antigen, the casein 3' untranslated region, or other 3' untranslated sequences well known in this art. Ribosome binding sites are also important in increasing the efficiency of expression of FIX. Likewise, sequences that regulate the post-translational modification of FIX are useful in the invention.

Factor IX coding sequences, along with vectors and host cells for the expression thereof, are disclosed in European Patent App. 373012, European Patent App. 251874, PCT Patent Appl. 8505376, PCT Patent Appln. 8505125, European Patent Appln. 162782, and PCT Patent Appln. 8400560.

### Compositions of the invention

Described herein is an isolated Factor IX (FIX) protein comprising at least three additional glycosylation sites relative to wild type human FIX, a K5R substitution and a R338X substitution of the FIX amino acid sequence of SEQ ID NO:1, wherein X is an amino acid other than arginine.
SEQ ID NO:1:

Described herein is an isolated Factor IX (FIX) protein comprising at least three additional glycosylation sites relative to wild type human FIX, a K51R substitution and a R384X substitution of the FIX amino acid sequence of SEQ ID NO:6, wherein X is an amino acid other than arginine.

Also described herein is a FIX protein comprising the amino acid sequence: wherein X is any amino acid except R (arginine).

Further described herein is a FIX protein comprising the amino acid sequence: wherein X is any amino acid except R (arginine).

The invention provides a FIX protein comprising the amino acid sequence: wherein Xaa is leucine.

Further described herein is an isolated nucleic acid molecule comprising the nucleotide sequence (FIX 24-K5R codon optimized sequence with propeptide sequence):

In further embodiments, the present invention provides an isolated nucleic acid molecule comprising the nucleotide sequence (FIX 24-K5R codon optimized sequence with propeptide sequence and a R338L substitution): wherein NNN is any three nucleotide codon encoding leucine.

The present invention also provides the use of the Factor IX protein, the nucleic acid molecule, the vector and/or the cell of this invention for treating a bleeding disorder.

Some embodiments of the invention are directed to Factor IX proteins having one or more (e.g., 1, 2, 3, 4, 5, 6, 7, 8, 9, 10, 11, 12, 13, 14, 15, etc.) additional glycosylation sites. By "additional" or "new" glycosylation sites is meant that the number of glycosylation sites in the FIX protein is greater than the number of glycosylation sites normally present in a "wild type" form of Factor IX. A Factor IX protein of this invention can include plasma derived FIX as well as recombinant forms of FIX. Generally, embodiments of the invention are directed to increasing the number of glycosylation sites in a FIX molecule of this invention. However, it is to be understood that a Factor IX protein of this invention that can be modified to increase the number of glycosylation sites and/or to increase the number of sugar chains is not limited to a particular "wild type" FIX amino acid sequence because naturally occurring or man-made FIX proteins can also be modified according to the methods of this invention to increase the number of glycosylation sites and/or to increase the number of sugar chains.

The present invention is further directed to FIX proteins containing one or more (e.g., 2, 3, 4, 5, 6, 7, 8, 9, 10, 11, 12, 13, 14, 15, etc.) additional sugar chains. Such additional sugar side chains can be present at one or more of the additional glycosylation sites introduced into the FIX proteins of this invention by the methods described herein. Alternatively, the additional sugar side chains can be present at sites on the FIX protein as a result of chemical and/or enzymatic methods to introduce such sugar chains to the FIX molecule, as are well known in the art. By "additional" or "new" sugar chains is meant that the number of sugar chains in the FIX protein is greater than the number of sugar chains normally present in a "wild type" form of Factor IX. In various embodiments, about 1 to about 50 additional sugar side chains (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) can be added.

In some embodiments, at least one additional glycosylation site is in the activation peptide of Factor IX (e.g., the human FIX activation peptide having the amino acid sequence of SEQ ID NO:1). Described herein, the FIX protein has an insertion of a peptide segment that introduces one or more glycosylation sites between position N157 and N167 of the Factor IX amino acid sequence of SEQ ID NO:1.

Insertion(s) can be introduced into a FIX protein described herein to increase the number of glycosylation sites and such insertion(s) can include from about one to about 100 amino acid residues, including any number of amino acid residues from one to 100 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100).

In some instances, the insertion can include all or at least part (e.g., at least 3, 4, 5, 6, 7, 8, 9, 10, 11, 12, 13, 14, 15 or more amino acid residues) of a Factor IX activation peptide from a non-human species, such as mouse. This inserted peptide sequence can be further modified to introduce additional glycosylation sites according to the teachings herein.

The glycosylation site(s) may be N-linked glycosylation site(s). In some embodiments, the added glycosylation site(s) include N-linked glycosylation site(s) and the consensus sequence is NXT/S, with the proviso that X is not proline.

In some embodiments about one to about 15 glycosylation site(s) can be added to the FIX amino acid sequence. In various embodiments, about 1 to about 50 glycosylation site(s) (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) can be added. Described herein are FIX proteins in which a glycosylation site has been created by insertion, deletion or substitution of specific amino acids. In particular instances, the insertion, deletion and/or substitution is in the region of the activation peptide. The amino acid sequence of the human FIX activation peptide is provided herein as: Ala Glu Thr Val Phe Pro Asp Val Asp Tyr Val Asn Ser Thr Glu Ala Glu Thr Ile Leu Asp Asn Ile Thr Gln Ser Thr Gln Ser Phe Asn Asp Phe Thr Arg (SEQ ID NO:11).

It is contemplated that the additional glycosylation sites introduced into a FIX amino acid sequence can be introduced anywhere throughout the amino acid sequence of the FIX protein. Thus, in some instances, the additional glycosylation site or sites are introduced in the activation peptide (amino acids 146-180 of the mature human FIX amino acid sequence of SEQ ID NO:1), outside the activation peptide (e.g., before and/or after the activation peptide) or both inside the activation peptide and outside the activation peptide. Thus, based on the numbering of the 415 amino acids of the amino acid sequence of the mature human FIX protein as shown in SEQ ID NO:1, a glycosylation attachment site can be introduced by inserting additional amino acid residues between any of amino acids 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122,123, 124, 125, 126, 127, 128, 129, 130, 131, 132, 133, 134, 135, 136, 137, 138, 139, 140,141, 142, 143, 144, 145, 146, 147, 148, 149, 150, 151, 152, 153, 154, 155,156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415 and any combination thereof. As used herein, a "glycosylation attachment site" or "glycosylation site" can mean a sugar attachment consensus sequence (i.e., a series of amino acids that act as a consensus sequence for attaching a sugar (mono-, oligo-, or poly-saccharide) to an amino acid sequence or it can mean the actual amino acid residue to which the sugar moiety is covalently linked. The sugar moiety can be a monosaccharide (simple sugar molecule), an oligosaccharide, or a polysaccharide.

In particular instances, additional amino acids can be inserted between and/or substituted into any of the amino acid residues that make up the activation peptide, such as between any of amino acids 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182 and any combination thereof. Furthermore, the same insert in this particular instance can be introduced multiple times at the same and/or at different locations in the amino acid sequence of the FIX protein, including within the activation peptide. Also, different inserts and/or the same inserts can be introduced one or more times at the same and/or at different locations between amino acid residues throughout the amino acid sequence of the FIX protein, including within the activation peptide.

It is well known in the art that some proteins can support a large number of sugar side chains and the distance between N-linked glycosylation sites can be as few as three, four, five or six amino acids (see, e.g., Lundin et al. "Membrane topology of the Drosophila OR83b odorant receptor" FEBS Letters 581:5601-5604 (2007); Apweiler et al. "On the frequency of protein glycosylation, as deduced from analysis of the SWISS-PROT database" Biochimica et Biophysica Acta 1473:4-8 (19991).

Furthermore, the FIX protein described herein can be modified by mutation (e.g., substitution, addition and/or deletion of amino acids) to introduce N-linked glycosylation sites. For example, amino acid residues on the surface of the functional FIX protein can be identified according to molecular modeling methods standard in the art that would be suitable for modification (e.g., mutation) to introduce one or more glycosylation sites.

FIX proteins described herein as having additional glycosylation sites may be produced by recombinant methods such as site-directed mutagenesis using PCR. Alternatively, the Factor IX protein of this invention may be chemically synthesized to prepare a Factor IX protein with one or more (e.g., 1, 2, 3, 4, 5, 6, 7, 8, 9, 10, 11, 12, 13, 14, 15, etc.) additional glycosylation sites.

It is within the skill of one of ordinary skill in the art to modify any amino acid residue or residues in the mature FIX amino acid sequence according to methods well known in the art and as taught herein and to test any resulting FIX protein for activity, stability, recovery, half life, etc., according to well known methods and as described herein (see, e.g., Elliott et al. "Structural requirements for additional N-linked carbohydrate on recombinant human erythropoietin" J. Biol. Chem. 279:16854-62 (2004).

Embodiments of the invention are directed to recombinant Factor IX proteins in which glycosylation sites have been added to improve the recovery and/or half-life and/or stability of Factor IX. The glycosylation sites may be N-linked glycosylation sites. In specific embodiments, at least one N-linked glycosylation site is added.

As noted herein, at least one additional glycosylation site is introduced into the FIX amino acid sequence at a site that is outside of the activation peptide. In some instances, the at least one additional glycosylation site corresponds to a site that is glycosylated in the native form of a non-human homolog of Factor IX. A modification of the human FIX amino acid sequence to introduce a serine or threonine at amino acid 262 of the amino acid sequence of SEQ ID NO:1, which is the mature (i.e., secreted) form of human FIX, would introduce an additional N-linked glycosylation site in the human protein. In various instances, the non-human homolog is from dog, pig, cow, or mouse.

Additionally provided herein is a nucleic acid comprising, consisting essentially of and/or consisting of a nucleotide sequence encoding a FIX amino acid sequence of this invention. Such nucleic acids can be present in a vector, such as an expression cassette. Thus, further embodiments of the invention are directed to expression cassettes designed to express a nucleotide sequence encoding any of the Factor IX proteins of this invention. The nucleic acids and/or vectors of t his invention can be present in a cell. Thus, various embodiments of the invention are directed to recombinant host cells containing the vector (e.g., expression cassette). Such a cell can be isolated and/or present in a non-human transgenic animal. Therefore, certain embodiments of the invention are further directed to a non-human transgenic animal comprising a nucleic acid comprising a nucleotide sequence encoding any of the Factor IX proteins of the present invention.

A comparison of the amino acid sequence of the activation peptide of human, mouse, guinea pig and platypus FIX reveals that the mouse FIX amino acid sequence has an additional nine amino acids present in its activation peptide, the guinea pig FIX amino acid sequence has an additional ten amino acid residues present in its activation peptide and the platypus has an additional 14 amino acid residues present in its activation peptide (Figure 5). These extra amino acids are between the two naturally occurring glycosylation sites (N 157 and N 167) in human Factor IX.

The human and mouse FIX have essentially identical structures and the human enzyme can function in the mouse. As the human FIX functions without the additional nine amino acid segment found in the mouse, this region of the Factor IX molecule can tolerate modifications within its sequence, including insertions, substitutions and/or deletions, without substantial loss in structural, biochemical, or otherwise functional integrity of the molecule. The inserted nine amino acids in mouse are most likely surface residues (as supported by structural studies) and therefore accessible for modification by the glycosylation enzymes. In native human factor IX, the two N-linked glycosylation sites are 12 and 14 amino acids distant from the amino and carboxyl cleavage sites, respectively, of the activation peptide. Thus, in some cases, additional amino acid residues can be added between N157 and N167 of the human Factor IX protein of SEQ ID NO:1 in order to add glycosylation sites to improve half life and/or bioavailability. In various cases, glycosylation sites are added by insertion, deletion and/or modification of the native sequence to include an attachment sequence for consensus sequences for N-linked glycosylation.

The human sequence for the activation peptide starts at residue 146 of the mature protein. The natural glycosylation sites are at N157 and N167 (SEQ ID NO:1). In some cases, additional amino acid residues can be inserted between the two normal glycosylation sites (between N157 and N167 in the mature sequence) to provide additional glycosylation sites. In some cases, about 3 to about 100 additional amino acid residues are added. In other cases, about 5 to about 50 amino acid residues are added. In further instances, about 5 to about 20 amino acid residues are added. In yet further instances, about 7 to about 15 amino acid residues are added. Typically, the amino acid residues are chosen from the 20 biological amino acids with the proviso that proline is not used as "X" in the glycosylation site NXT/S, which is the consensus sequence for N-linked glycosylation. **Table 1** shows 20 common biological amino acids and their abbreviations.

N-glycosylation sites may be added. Consensus sequences for addition of glycosylation sites are known in the art and include the consensus sequence "NXT/S" for N-glycosylation where X is not proline.

In some cases, endogenous N-linked attachment sequences from mouse, human and other mammalian Factor IX sequences are inserted into the activation peptide. These may be inserted individually or in combination. In certain instances, the inserted segment includes a spacer region between glycosylation sites, which can be present individually, in tandem repeats, in multiples, etc. A spacer region described herein can be from one to about 100 amino acids in length (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and 100). In some cases, for example, the spacer region can be from one to about 20 amino acids. In other cases the spacer region can be from one to about ten amino acids. In further cases, the spacer region can be from one to about five amino acid residues.

A spacer region described herein is included between the added carbohydrate attachment sites and/or between naturally occurring glycosylation sites and added glycosylation sites to reduce or eliminate steric hindrance and provide efficient recognition by the appropriate glycosyltransferase. A spacer region described herein can be comprised of any combination of amino acid residues provided that they are not cysteine or proline and provided that the amino acid sequence of the spacer does not have more than about 10% residues that are hydrophobic (e.g., tryptophan, tyrosine, phenylalanine and valine).

In some instances, NXT/S is incorporated into the inserted amino acid sequence to add one or more additional glycosylation sites. "X" may be any biological amino acid except that proline is disfavored. In some embodiments, at least one additional glycosylation site is added to the Factor IX protein. In other embodiments, two additional glycosylation sites are added. In further embodiments, three additional glycosylation sites are added. In yet further embodiments, four additional glycosylation sites are added. In further embodiments, five additional glycosylation sites are added. In some embodiments, six additional glycosylation sites are added. In other embodiments, more than six additional glycosylation sites are added.

In one instance, Ala at position 161 of the mature FIX amino acid sequence (SEQ ID NO: 1) is replaced with Asn to provide one additional glycosylation site. In a further instance, the sequence VFIQDNITD (SEQ ID NO:8) is inserted between residues 161 and 162 of the mature human FIX amino acid sequence of SEQ ID NO:1 to introduce an N-linked glycosylation site in the human FIX sequence. In yet a further instance, another new glycosylation site is added by replacing Asp with Asn in the VFIQDNITD insert. The inserted sequence would give VFIQDNITN (SEQ ID NO:9). The instances discussed above could be combined to provide one to four additional glycosylation sites in the human Factor IX protein.

In another embodiment, the following sequence is added, which provides five additional glycosylation sites. The glycosylation sites are shown in bold and underlined. AETVFPDVDYV**N**STE**N**ETIQD**N**ITD**N**ETILD**N**ITQSTQSFNDFTR (SEQ ID NO: 10)

In some embodiments, glycosylation sites are added at sites outside of the activation peptide. These additional sites can be selected, for example, by aligning the amino acid sequence of Factor IX from human with the Factor IX amino acid sequence from other species and determining the position of glycosylation sites in non-human species. The homologous or equivalent position in the human FIX amino acid sequence is then modified to provide a glycosylation site. This method may be used to identify both potential N-glycosylation and O-glycosylation sites.

The FIX proteins according to the invention are produced and characterized by methods well known in the art and as described herein. These methods include determination of clotting time (partial thromboplastin time (PPT) assay) and administration of the FIX protein to a test animal to determine recovery, half life, and bioavailability by an appropriate immunoassay and/or activity-assay, as are well known in the art.

The Factor IX protein, nucleic acid, vector and/or cell of this invention can be included in a pharmaceutical composition. Some embodiments are directed to a kit which includes the Factor IX protein of this invention. The Factor IX protein of this invention can be used for treating a bleeding disorder by administering an effective amount of the Factor IX protein to a subject (e.g., a human patient) in need thereof. Thus, the present invention also provides the use of the Factor IX protein, the nucleic acid molecule, the vector and/or the cell of this invention for treating a bleeding disorder. Bleeding disorders that can be treated according to the uses of this invention include a FIX deficiency, hemophilia B and Christmas disease. Such treatment protocols and dosing regimens for administering or delivering Factor IX to a subject in need thereof are well known in the art.

Many expression vectors can be used to create genetically engineered cells. Some expression vectors are designed to express large quantities of recombinant proteins after amplification of transfected cells under a variety of conditions that favor selected, high expressing cells. Some expression vectors are designed to express large quantities of recombinant proteins without the need for amplification under selection pressure. The present invention includes the production of genetically engineered cells according to methods standard in the art and is not dependent on the use of any specific expression vector or expression system.

To create a genetically engineered cell to produce large quantities of a Factor IX protein, cells are transfected with an expression vector that contains the cDNA encoding the protein. In some embodiments, the target protein is expressed with selected co-transfected enzymes that cause proper post-translational modification of the target protein to occur in a given cell system.

The cell may be selected from a variety of sources, but is otherwise a cell that may be transfected with an expression vector containing a nucleic acid, preferably a cDNA encoding a Factor IX protein.

The practice of the present invention employs, unless otherwise indicated, conventional techniques of molecular biology, microbiology, recombinant DNA, and immunology, which are within the skill of the art. Such techniques are explained fully in the literature. See, e.g., Sambrook, et al., Molecular Cloning; A Laboratory Manual, 2nd ed. (1989); DNA Cloning, Vols. I and II (D. N Glover, ed. 1985); Oligonucleotide Synthesis (M. J. Gait, ed. 1984); Nucleic Acid Hybridization (B. D. Hames & S. J. Higgins, eds. 1984); Transcription and Translation (B. D. Hames & S. J. Higgins, eds. 1984); Animal Cell Culture (R. I. Freshney, ed. 1986); Immobilized Cells and Enzymes (IRL Press, 1986); B. Perbal, A Practical Guide to Molecular Cloning (1984); the series, Methods in Enzymology (Academic Press, Inc.), particularly Vols. 154 and 155 (Wu and Grossman, and Wu, eds., respectively); Gene Transfer Vectors for Mammalian Cells (J. H. Miller and M. P. Calos, eds. 1987, Cold Spring Harbor Laboratory); Immunochemical Methods in Cell and Molecular Biology, Mayer and Walker, eds. (Academic Press, London, 1987); Scopes, Protein Purification: Principles and Practice, 2nd ed. 1987 (Springer-Verlag, N.Y.); and Handbook of Experimental Immunology Vols I-IV (D. M. Weir and C. C. Blackwell, eds 1986).

### Genetic Engineering Techniques

The production of cloned genes, recombinant DNA, vectors, transformed host cells, proteins and protein fragments by genetic engineering is well known. See, e.g., U.S. Pat. No. 4,761,371 to Bell et al. at Col. 6, line 3 to Col. 9, line 65; U.S. Pat. No. 4,877,729 to Clark et al. at Col. 4, line 38 to Col. 7, line 6; U.S. Pat. No. 4,912,038 to Schilling at Col. 3, line 26 to Col. 14, line 12; and U.S. Pat. No. 4,879,224 to Wallner at Col. 6, line 8 to Col. 8, line 59.

A vector is a replicable DNA construct. Vectors are used herein either to amplify nucleic acid encoding Factor IX protein and/or to express nucleic acid which encodes Factor IX protein. An expression vector is a replicable nucleic acid construct in which a nucleotide sequence encoding a Factor IX protein is operably linked to suitable control sequences capable of effecting the expression of the nucleotide sequence to produce a Factor IX protein in a suitable host. The need for such control sequences will vary depending upon the host selected and the transformation method chosen. Generally, control sequences include a transcriptional promoter, an optional operator sequence to control transcription, a sequence encoding suitable mRNA ribosomal binding sites, and sequences that control the termination of transcription and translation.

Vectors comprise plasmids, viruses (e.g., adenovirus, cytomegalovirus), phage, and integratable DNA fragments (i.e., fragments integratable into the host genome by recombination). The vector replicates and functions independently of the host genome, or may, in some instances, integrate into the genome itself. Expression vectors can contain a promoter and RNA binding sites that are operably linked to the gene to be expressed and are operable in the host organism.

DNA regions or nucleotide sequences are operably linked or operably associated when they are functionally related to each other. For example, a promoter is operably linked to a coding sequence if it controls the transcription of the sequence; or a ribosome binding site is operably linked to a coding sequence if it is positioned so as to permit translation of the sequence.

Transformed host cells are cells which have been transformed, transduced and/or transfected with Factor IX protein vector(s) constructed using recombinant DNA techniques.

Suitable host cells include prokaryote, yeast or higher eukaryotic cells such as mammalian cells and insect cells. Cells derived from multicellular organisms are a particularly suitable host for recombinant Factor IX protein synthesis, and mammalian cells are particularly preferred. Propagation of such cells in cell culture has become a routine procedure (Tissue Culture, Academic Press, Kruse and Patterson, editors (1973)). Examples of useful host cell lines are VERO and HeLa cells, Chinese hamster ovary (CHO) cell lines, and WI138, HEK 293, BHK, COS-7, CV, and MDCK cell lines. Expression vectors for such cells ordinarily include (if necessary) an origin of replication, a promoter located upstream from the nucleotide sequence encoding Factor IX protein to be expressed and operatively associated therewith, along with a ribosome binding site, an RNA splice site (if intron-containing genomic DNA is used), a polyadenylation site, and a transcriptional termination sequence. In a preferred embodiment, expression is carried out in Chinese Hamster Ovary (CHO) cells using the expression system of U.S. Patent No. 5,888,809.

The transcriptional and translational control sequences in expression vectors to be used in transforming vertebrate cells are often provided by viral sources. For example, commonly used promoters are derived from polyoma, Adenovirus 2, and Simian Virus 40 (SV40). See. e.g., U.S. Pat. No. 4,599,308.

An origin of replication may be provided either by construction of the vector to include an exogenous origin, such as may be derived from SV 40 or other viral (e.g., polyoma, adenovirus, VSV, or BPV) source, or may be provided by the host cell chromosomal replication mechanism. If the vector is integrated into the host cell chromosome, the latter is often sufficient.

Rather than using vectors which contain viral origins of replication, one can transform mammalian cells by the method of cotransformation with a selectable marker and the nucleic acid encoding the Factor IX protein. Examples of suitable selectable markers are dihydrofolate reductase (DHFR) or thymidine kinase. This method is further described in U.S. Pat. No. 4,399,216.

Other methods suitable for adaptation to the synthesis of Factor IX protein in recombinant vertebrate cell culture include those described in Gething et al. Nature 293:620 (1981); Mantei et al. Nature 281:40; and Levinson et al., EPO Application Nos. 117,060A and 117,058A.

Host cells such as insect cells (e.g., cultured *Spodoptera frugiperda* cells) and expression vectors such as the baculovirus expression vector (e.g., vectors derived from *Autographa californica* MNPV, *Trichoplusia ni* MNPV, *Rachiplusia ou* MNPV, or *Galleria ou* MNPV) may be employed in carrying out the present invention, as described in U.S. Pat. Nos. 4,745,051 and 4,879,236 to Smith et al. In general, a baculovirus expression vector comprises a baculovirus genome containing the nucleotide sequence to be expressed inserted into the polyhedrin gene at a position ranging from the polyhedrin transcriptional start signal to the ATG start site and under the transcriptional control of a baculovirus polyhedrin promoter.

Prokaryote host cells include gram negative or gram positive organisms, for example *Escherichia coli* (*E. coli*) or bacilli. Higher eukaryotic cells include established cell lines of mammalian origin as described below. Exemplary host cells are *E. coli* W3110 (ATCC 27,325), *E. coli* B, *E. coli* X1776 (ATCC 31,537) and *E. coli* 294 (ATCC 31,446). A broad variety of suitable prokaryotic and microbial vectors are available. *E. coli* is typically transformed using pBR322. Promoters most commonly used in recombinant microbial expression vectors include the betalactamase (penicillinase) and lactose promoter systems (Chang et al. Nature 275:615 (1978); and Goeddel et al. Nature 281:544 (1979)), a tryptophan (trp) promoter system (Goeddel et al. Nucleic Acids Res. 8:4057 (1980) and EPO App. Publ. No. 36,776) and the tac promoter (De Boer et al. *Proc. Natl. Acad. Sci.* USA 80:21 (1983)). The promoter and Shine-Dalgarno sequence (for prokaryotic host expression) are operably linked to the nucleic acid encoding the Factor IX protein, i.e., they are positioned so as to promote transcription of Factor IX messenger RNA from DNA.

Eukaryotic microbes such as yeast cultures may also be transformed with protein-encoding vectors (see, e.g., U.S. Pat. No. 4,745,057). *Saccharomyces cerevisiae* is the most commonly used among lower eukaryotic host microorganisms, although a number of other strains are commonly available. Yeast vectors may contain an origin of replication from the 2 micron yeast plasmid or an autonomously replicating sequence (ARS), a promoter, nucleic acid encoding Factor IX protein, sequences for polyadenylation and transcription termination, and a selection gene. An exemplary plasmid is YRp7, (Stinchcomb et al. Nature 282:39 (1979); Kingsman et al. Gene 7:141 (1979); Tschemper et al. Gene 10:157 (1980)). Suitable promoting sequences in yeast vectors include the promoters for metallothionein, 3-phosphoglycerate kinase (Hitzeman et al. J. Biol. Chem. 255:2073 (1980) or other glycolytic enzymes (Hess et al. J. Adv. Enzyme Reg. 7:149 (1968); and Holland et al. Biochemistry 17:4900 (1978)). Suitable vectors and promoters for use in yeast expression are further described in R. Hitzeman et al., EPO Publn. No. 73,657.

Cloned coding sequences described herein may encode FIX of any species of origin, including mouse, rat, dog, opossum, rabbit, cat, pig, horse, sheep, cow, guinea pig, opossum, platypus, and human, but preferably encode Factor IX protein of human origin. Nucleic acid encoding Factor IX that is hybridizable with nucleic acid encoding proteins disclosed herein is also encompassed. Hybridization of such sequences may be carried out under conditions of reduced stringency or even stringent conditions (e.g., stringent conditions as represented by a wash stringency of 0.3M NaCl, 0.03M sodium citrate, 0.1% SDS at 60°C or even 70°C) to nucleic acid encoding Factor IX protein disclosed herein in a standard *in situ* hybridization assay. See, e.g., Sambrook et al., Molecular Cloning, A Laboratory Manual (2d Ed. 1989) Cold Spring Harbor Laboratory).

The FIX proteins produced according to the invention may be expressed in non-human transgenic animals by known methods. See for example, U.S. Patent No. 6,344,596. In brief, non-human transgenic animals may include but are not limited to farm animals (e.g., pigs, goats, sheep, cows, horses, rabbits and the like) rodents (such as mice, rats and guinea pigs), and domestic pets (for example, cats and dogs). Livestock animals such as pigs, sheep, goats and cows, are particularly preferred in some embodiments.

The non-human transgenic animal of this invention is produced by introducing into a single cell non-human embryo an appropriate polynucleotide that encodes a human Factor IX protein of this invention in a manner such that the polynucleotide is stably integrated into the DNA of germ line cells of the mature animal, and is inherited in normal Mendelian fashion. The non-human transgenic animal of this invention would have a phenotype of producing the FIX protein in body fluids and/or tissues. The FIX protein would be removed from these fluids and/or tissues and processed, for example for therapeutic use. (See, e.g., Clark et al. "Expression of human anti-hemophilic factor IX in the milk of transgenic sheep" Bio/Technology 7:487-492 (1989); Van Cott et al. "Haemophilic factors produced by transgenic livestock: abundance can enable alternative therapies worldwide" Haemophilia 10(4):70-77 (2004).

DNA molecules can be introduced into non-human embryos by a variety of means including but not limited to microinjection, calcium phosphate mediated precipitation, liposome fusion, or retroviral infection of totipotent or pluripotent stem cells. The transformed cells can then be introduced into non-human embryos and incorporated therein to form non-human transgenic animals. Methods of making non-human transgenic animals are described, for example, in Transgenic Animal Generation and Use by L. M. Houdebine, Harwood Academic Press, 1997. Transgenic animals also can be generated using methods of nuclear transfer or cloning using embryonic or adult cell lines as described for example in Campbell et al., Nature 380:64-66 (1996) and Wilmut et al., Nature 385:810-813 (1997). Further a technique utilizing cytoplasmic injection of DNA can be used as described in U.S. Pat. No. 5,523,222.

Factor IX-producing non-human transgenic animals can be obtained by introducing a chimeric construct comprising Factor IX-encoding sequences. Methods for obtaining transgenic animals are well-known. See, for example, Hogan et al., MANIPULATING THE MOUSE EMBRYO, (Cold Spring Harbor Press 1986); Krimpenfort et al., Bio/Technology 9:88 (1991); Palmiter et al., Cell 41:343 (1985), Kraemer et al., GENETIC MANIPULATION OF THE EARLY MAMMALIAN EMBRYO, (Cold Spring Harbor Laboratory Press 1985); Hammer et al., Nature 315:680 (1985); Wagner et al., U.S. Pat. No. 5,175,385; Krimpenfort et al., U.S. Pat. No. 5,175,384, Janne et al., Ann. Med. 24:273 (1992), Brem et al., Chim. Oggi. 11:21 (1993), Clark et al., U.S. Pat. No. 5,476,995.

In some embodiments, cis-acting regulatory regions may be used that are "active" in mammary tissue in that the promoters are more active in mammary tissue than in other tissues under physiological conditions where milk is synthesized. Such promoters include but are not limited to the short and long whey acidic protein (WAP), short and long α, β and κ casein, α-lactalbumin and β-lactoglobulin ("BLG") promoters. Signal sequences can also be used in accordance with this invention that direct the secretion of expressed proteins into other body fluids, particularly blood and urine. Examples of such sequences include the signal peptides of secreted coagulation factors including signal peptides of Factor IX, protein C, and tissue-type plasminogen activator.

Among the useful sequences that regulate transcription, in addition to the promoters discussed above, are enhancers, splice signals, transcription termination signals, polyadenylation sites, buffering sequences, RNA processing sequences and other sequences which regulate the expression of transgenes.

Preferably, the expression system or construct includes a 3' untranslated region downstream of the nucleotide sequence encoding the desired recombinant protein. This region can increase expression of the transgene. Among the 3' untranslated regions useful in this regard are sequences that provide a poly A signal.

Suitable heterologous 3'-untranslated sequences can be derived, for example, from the SV40 small t antigen, the casein 3' untranslated region, or other 3' untranslated sequences well known in this art. Ribosome binding sites are also important in increasing the efficiency of expression of FIX. Likewise, sequences that regulate the post-translational modification of FIX are useful in the invention.

Factor IX coding sequences, along with vectors and host cells for the expression thereof, are disclosed in European Patent App. 373012, European Patent App. 251874, PCT Patent Appl. 8505376, PCT Patent Appln. 8505125, European Patent Appln. 162782, and PCT Patent Appln. 8400560.

### EXAMPLES

### Example 1. (Comparative Example)

***Abstract.*** Factor IX (FIX) has an unusual half-life; around 50-70% disappears from the circulation within ∼ 5 minutes of injection. Practically, the half-life is calculated from the second exponential decay of the FIX remaining in circulation. This study shows that FIX protects hemophilia B mice from bleeding well after its blood levels are below 1%. This protective effect is believed to be due to FIX binding specifically and reversibly to type IV collagen and still being available for coagulation even though it has disappeared from the blood. Consistent with this, K5RFIX, which binds tighter to type IV collagen than does K5AFIX, protects better 7 days after injection than does K5AFIX.

This study demonstrates a correlation between the affinity of FIX for type IV collagen and its ability to protect hemophilia B mice from bleeding by a saphenous vein bleeding model.

**Saphenous vein bleeding model.** Six to eight-week-old hemophilia B mice on a C57BL/6 background were used in this study. FIX variant proteins were injected into mice via tail vein with a dose of 0.9 ug/g body weight. After 7 days, the saphenous vein bleeding model was performed. Briefly, mice were anesthetized with 2.5% Avertin and a longitudinal incision was made on the left saphenous vein. Blood was gently wicked away with a tissue until hemostasis occurred. The clot was then disrupted and blood wicked away again until hemostasis occurred. Clot disruption was repeated after every incidence of hemostasis for 30 minutes following the initial injury. The number of clot disruptions observed for each mouse was recorded.

The data provided herein show that, not only does K5RFIX protect for longer than one might expect from its half-life but that WTFIX also protects hemophilia B mice from bleeding for much longer than one would expect from its observed half-life. **Figure 1** shows that mice injected with human FIX are still significantly protected from bleeding seven days after injection. The clotting potential is assessed by measuring the number of clot disruptions for each mouse. This protection is present despite the fact that the half-life of human FIX in hemophilia B mice is about 7 hours; this means that 24 half-lives have elapsed since injection, and that the level of circulating FIX was below 1% since ∼day 2.5.

Moreover, in **Figure 1** it can be seen that after 7 days, K5RFIX protects hemophilia mice from bleeding significantly better than K5AFIX (*P <0.05*, unpaired t-test). K5RFIX binds type IV collagen tighter than does WTFIX while K5AFIX has a much reduced affinity to collagen.

In summary, the amount of FIX measured in blood is much less than the total amount of FIX available for coagulation. Also, FIX protects from bleeding much longer than one would expect based on its measured half-life.

### Example 2.

The DNA sequence of codon optimized FIX 24 with extra glycosylation sites was synthesized by Blueheron Biotech, LLC. The synthesized sequence was inserted into pDEF38 vector and transfected in CHO DG44 cells. Single clones were picked by CDI Bioscience, Inc. Conditioned medium was collected from these clones and examined by Western blot with anti-FIX antibody.

As shown in **Figure 2**, with the addition of more glycosylation sites, the size of FIX shifts up. FIX 24 has the highest molecular weight. The modified Factor IX proteins of this invention will be used in half life studies in mice and in dogs, according to known protocols. The Factor IX proteins of this invention will also be tested in a saphenous vein model study in mice for longer time periods (e.g., 3 weeks) to determine how long the proteins protect the mice. The rate of activation of the modified Factor IX proteins will be determined by FVIIa-TF and by factor Xia. Specific activities will also be determined, as well as the state of glycosylation and carboxylation, according to known methods.

### Example 3.

***Abstract.*** Factor IX (FIX) has an unusual half-life; around 50-70% disappears from the circulation within ∼ 5 minutes of injection. Practically, the half-life is calculated from the second exponential decay of the FIX remaining in circulation. This study shows that FIX protects hemophilia B mice from bleeding well after its blood levels are below 1%. This protective effect is believed to be due to FIX binding specifically and reversibly to type IV collagen and still being available for coagulation even though it has disappeared from the blood. Consistent with this, K5RFIX, which binds tighter to type IV collagen than does K5AFIX, protects better 7 days after injection than does K5AFIX.

This study demonstrates a correlation between the affinity of FIX for type IV collagen and its ability to protect hemophilia B mice from bleeding by a saphenous vein bleeding model.

**Saphenous vein bleeding model.** Six to eight-week-old hemophilia B mice on a C57BL/6 background were used in this study. FIX variant proteins were injected into mice via tail vein with a dose of 0.9 ug/g body weight. After 7 days, the saphenous vein bleeding model was performed. Briefly, mice were anesthetized with 2.5% Avertin and a longitudinal incision was made on the left saphenous vein. Blood was gently wicked away with a tissue until hemostasis occurred. The clot was then disrupted and blood wicked away again until hemostasis occurred. Clot disruption was repeated after every incidence of hemostasis for 30 minutes following the initial injury. The number of clot disruptions observed for each mouse was recorded.

The data provided herein show that, not only does K5RFIX protect for longer than one might expect from its half-life but that WTFIX also protects hemophilia B mice from bleeding for much longer than one would expect from its observed half-life. **Figure 3** shows that mice injected with human FIX are still significantly protected from bleeding seven days after injection. The clotting potential is assessed by measuring the number of clot disruptions for each mouse. This protection is present despite the fact that the half-life of human FIX in hemophilia B mice is about 7 hours; this means that 24 half-lives have elapsed since injection, and that the level of circulating FIX was below 1% since ∼day 2.5.

Moreover, in **Figure 3** it can be seen that after 7 days, K5RFIX protects hemophilia mice from bleeding significantly better than K5AFIX (P *<0.05,* unpaired t-test). K5RFIX binds type IV collagen tighter than does WTFIX while K5AFIX has a much reduced affinity to collagen.

In summary, the amount of FIX measured in blood is much less than the total amount of FIX available for coagulation. Also, FIX protects from bleeding much longer than one would expect based on its measured half-life.

The studies described herein were designed to test whether infused FIX might protect hemophilia B mice from bleeding longer than expected based on half-life and whether FIX_{K5R} protects better than FIX_{K5A}. **Figure 3** reveals that infused FIX protects much longer than would be predicted by its' half-life; thus, there is good protection 7 days after injection--even though the plasma levels of all of the infused FIX molecules were below one percent by day 3 after infusion. These results demonstrate that extravascular, collagen IV-bound FIX provides an important coagulant function in the absence of circulating FIX. Moreover, there is a clear gradient of protection which correlates to the affinity of the molecules for collagen IV.

For FIX, the terminal half-life (β) is usually considered the relevant parameter, while the distribution half-life (α) is ignored. The goal of prophylaxis in patients with severe hemophilia B is to maintain trough levels of FIX activity in the circulation above 1%.

In conclusion, evidence is provided herein that FIX effectively prevents bleeding even after its blood level has been well below one percent for several days. In addition, seven days after a bolus infusion, a FIX variant that binds tighter to collagen IV provides significantly better hemostatic protection in hemophilia B mice than a FIX molecule with lower affinity for collagen IV. This demonstrates that collagen IV-binding by FIX provides a longer lasting extravascular reservoir of FIX at a hemostatically functional location. Furthermore, these results suggest that a therapeutic focus limited to increasing the terminal plasma half-life of FIX alone at the expense of its' tissue distribution may not be the optimal approach for the treatment of hemophilia B.

### Example 4.

The DNA sequence of codon optimized FIX 24 with extra glycosylation sites was synthesized by Blueheron Biotech, LLC. The synthesized sequence was inserted into pDEF38 vector and transfected in CHO DG44 cells. Single clones were picked by CDI Bioscience, Inc. Conditioned medium was collected from these clones and examined by Western blot with anti-FIX antibody.

With the addition of more glycosylation sites, the size of FIX shifts up. The modified Factor IX proteins of this invention will be used in half life studies in mice and in dogs, according to known protocols. The Factor IX proteins of this invention will also be tested in a saphenous vein model study in mice for longer time periods (e.g., 3 weeks) to determine how long the proteins protect the mice. The rate of activation of the modified Factor IX proteins will be determined by FVIIa-TF and by factor Xia. Specific activities will also be determined, as well as the state of glycosylation and carboxylation, according to known methods.

The invention is defined by the following claims, with equivalents of the claims to be included therein.

**Table 1**

| **Amino Acids** | **One-Letter Symbol** | **Common Abbreviation** |
|---|---|---|
| Alanine | A | Ala |
| Arginine | R | Arg |
| Asparagine | N | Asn |
| Aspartic acid | D | Asp |
| Cysteine | C | Cys |
| Glutamine | Q | Gln |
| Glutamic acid | E | Glu |
| Glycine | G | Gly |
| Histidine | H | His |
| Isoleucine | I | Ile |
| Leucine | L | Leu |
| Lysine | K | Lys |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |

## Claims

1. An isolated Factor IX (FIX) protein comprising the amino acid sequence as set forth in the SEQ ID NO: 3, wherein Xaa is leucine.

2. An isolated nucleic acid molecule encoding the isolated FIX protein of claim 1.

3. The isolated nucleic acid molecule of claim 2, wherein the nucleic acid molecule comprises the sequence as set forth in SEQ ID NO: 5

4. A vector or transformed cell comprising the nucleic acid of claim 2.

5. A non-human transgenic animal comprising the nucleic acid of claim 2 or the vector or transformed cell of claim 4.

6. The isolated Factor IX protein of claim 1 for use in treating a bleeding disorder, optionally selected from the group consisting of a FIX deficiency, hemophilia B and Christmas disease.

7. The isolated nucleic acid molecule of claim 2 for use in treating a bleeding disorder, optionally selected from the group consisting of a FIX deficiency, hemophilia B and Christmas disease.

8. The vector or transformed cell of claim 4 for use in treating a bleeding disorder, optionally selected from the group consisting of a FIX deficiency, hemophilia B and Christmas disease.

## Patentansprüche

1. Ein isoliertes Faktor IX (FIX) Protein, umfassend die Aminosäuresequenz wie in SEQ ID NO:3 dargestellt, wobei Xaa Leucin ist.

2. Ein isoliertes Nukleinsäuremolekül, kodierend das isolierte FIX Protein nach Anspruch 1.

3. Das isolierte Nukleinsäuremolekül nach Anspruch 2, wobei das Nukleinsäuremolekül die Sequenz wie in SEQ ID NO:5 dargestellt umfasst.

4. Ein Vektor oder eine transfomierte Zelle, umfassend die Nukleinsäure nach Anspruch 2.

5. Ein nicht-menschliches transgenes Tier, umfassend die Nukleinsäure nach Anspruch 2 oder den Vektor oder die transformierte Zelle nach Anspruch 4.

6. Das isolierte Faktor IX Protein nach Anspruch 1 zur Verwendung in der Behandlung einer Bluter-Erkrankung, optional ausgewählt aus der Gruppe bestehend aus einem FIX Mangel, Hämophilie B und der Christmas Erkrankung.

7. Das isolierte Nukleinsäuremolekül nach Anspruch 2 zur Verwendung in der Behandlung einer Bluter-Erkrankung optional ausgewählt aus der Gruppe bestehend aus einem FIX Mangel, Hämophilie B und der Christmas Erkrankung.

8. Der Vektor oder die transformierte Zelle nach Anspruch 4 zur Verwendung in der Behandlung einer Bluter-Erkrankung optional ausgewählt aus der Gruppe bestehend aus einem FIX Mangel, Hämophilie B und der Christmas Erkrankung.

## Revendications

1. Protéine Facteur IX (FIX) isolée comprenant la séquence d'acides aminés telle qu'énoncée dans la SEQ ID NO: 3, dans laquelle Xaa est la leucine.

2. Molécule d'acide nucléique isolée codant pour la protéine FIX isolée de la revendication 1.

3. Molécule d'acide nucléique isolée selon la revendication 2, dans laquelle la molécule d'acide nucléique comprend la séquence telle qu'énoncée dans SEQ ID NO: 5.

4. Vecteur ou cellule transformée comprenant l'acide nucléique de la revendication 2.

5. Animal transgénique non humain comprenant l'acide nucléique selon la revendication 2 ou le vecteur ou la cellule transformée de la revendication 4.

6. Protéine Facteur IX isolée selon la revendication 1 pour une utilisation dans le traitement d'un trouble hémorragique, facultativement choisi dans le groupe consistant en une déficience en FIX, l'hémophilie B et la maladie de Christmas.

7. Molécule d'acide nucléique isolée selon la revendication 2 pour une utilisation dans le traitement d'un trouble hémorragique, facultativement choisi dans le groupe consistant en une déficience en FIX, l'hémophilie B et la maladie de Christmas.

8. Vecteur ou cellule transformée selon la revendication 4 pour une utilisation dans le traitement d'un trouble hémorragique, facultativement choisi dans le groupe consistant en une déficience en FIX, l'hémophilie B et la maladie de Christmas.


### SEQUENCE LISTING

<110> University of North Carolina at Chapel Hill
<120> METHODS AND COMPOSITIONS FOR MODIFIED FACTOR IX PROTEINS
<130> 5470-643WO
<150> US 61/879,394
   <151> 2013-09-18
<150> US 61/728,469
   <151> 2012-11-20
<160> 11
<170> PatentIn version 3.5
<210> 1
   <211> 415
   <212> PRT
   <213> Homo sapiens
<400> 1
   Tyr Asn Ser Gly Lys Leu Glu Glu Phe Val Gln Gly Asn Leu Glu Arg
   1               5                   10                  15
   Glu Cys Met Glu Glu Lys Cys Ser Phe Glu Glu Ala Arg Glu Val Phe
               20                  25                  30
   Glu Asn Thr Glu Arg Thr Thr Glu Phe Trp Lys Gln Tyr Val Asp Gly
           35                  40                  45
   Asp Gln Cys Glu Ser Asn Pro Cys Leu Asn Gly Gly Ser Cys Lys Asp
       50                  55                  60
   Asp Ile Asn Ser Tyr Glu Cys Trp Cys Pro Phe Gly Phe Glu Gly Lys
   65                  70                  75                  80
   Asn Cys Glu Leu Asp Val Thr Cys Asn Ile Lys Asn Gly Arg Cys Glu
                   85                  90                  95
   Gln Phe Cys Lys Asn Ser Ala Asp Asn Lys Val Val Cys Ser Cys Thr
               100                 105                 110
   Glu Gly Tyr Arg Leu Ala Glu Asn Gln Lys Ser Cys Glu Pro Ala Val
           115                 120                 125
   Pro Phe Pro Cys Gly Arg Val Ser Val Ser Gln Thr Ser Lys Leu Thr
       130                 135                 140
   Arg Ala Glu Thr Val Phe Pro Asp Val Asp Tyr Val Asn Ser Thr Glu
   145                 150                 155                 160
   Ala Glu Thr Ile Leu Asp Asn Ile Thr Gln Ser Thr Gln Ser Phe Asn
                   165                 170                 175
   Asp Phe Thr Arg Val Val Gly Gly Glu Asp Ala Lys Pro Gly Gln Phe
               180                 185                 190
   Pro Trp Gln Val Val Leu Asn Gly Lys Val Asp Ala Phe Cys Gly Gly
           195                 200                 205
   Ser Ile Val Asn Glu Lys Trp Ile Val Thr Ala Ala His Cys Val Glu
       210                 215                 220
   Thr Gly Val Lys Ile Thr Val Val Ala Gly Glu His Asn Ile Glu Glu
   225                 230                 235                 240
   Thr Glu His Thr Glu Gln Lys Arg Asn Val Ile Arg Ile Ile Pro His
                   245                 250                 255
   His Asn Tyr Asn Ala Ala Ile Asn Lys Tyr Asn His Asp Ile Ala Leu
               260                 265                 270
   Leu Glu Leu Asp Glu Pro Leu Val Leu Asn Ser Tyr Val Thr Pro Ile
           275                 280                 285
   Cys Ile Ala Asp Lys Glu Tyr Thr Asn Ile Phe Leu Lys Phe Gly Ser
       290                 295                 300
   Gly Tyr Val Ser Gly Trp Gly Arg Val Phe His Lys Gly Arg Ser Ala
   305                 310                 315                 320
   Leu Val Leu Gln Tyr Leu Arg Val Pro Leu Val Asp Arg Ala Thr Cys
                   325                 330                 335
   Leu Arg Ser Thr Lys Phe Thr Ile Tyr Asn Asn Met Phe Cys Ala Gly
               340                 345                 350
   Phe His Glu Gly Gly Arg Asp Ser Cys Gln Gly Asp Ser Gly Gly Pro
           355                 360                 365
   His Val Thr Glu Val Glu Gly Thr Ser Phe Leu Thr Gly Ile Ile Ser
       370                 375                 380
   Trp Gly Glu Glu Cys Ala Met Lys Gly Lys Tyr Gly Ile Tyr Thr Lys
   385                 390                 395                 400
   Val Ser Arg Tyr Val Asn Trp Ile Lys Glu Lys Thr Lys Leu Thr
   405 410 415
<210> 2
   <211> 465
   <212> PRT
   <213> Artificial
<220>
   <223> Modified Factor IX protein sequence
<220>
   <221> misc_feature
   <222> (388)..(388)
   <223> Xaa can be any naturally occurring amino acid
<400> 2
   Tyr Asn Ser Gly Arg Leu Glu Glu Phe Val Gln Gly Asn Leu Glu Arg
   1               5                   10                  15
   Glu Cys Met Glu Glu Lys Cys Ser Phe Glu Glu Ala Arg Glu Val Phe
               20                  25                  30
   Glu Asn Thr Glu Arg Thr Thr Glu Phe Trp Lys Gln Tyr Val Asp Gly
           35                  40                  45
   Asp Gln Cys Glu Ser Asn Pro Cys Leu Asn Gly Gly Ser Cys Lys Asp
       50                  55                  60
   Asp Ile Asn Ser Tyr Glu Cys Trp Cys Pro Phe Gly Phe Glu Gly Lys
   65                  70                  75                  80
   Asn Cys Glu Leu Asp Val Thr Cys Asn Ile Lys Asn Gly Arg Cys Glu
                   85                  90                  95
   Gln Phe Cys Lys Asn Ser Ala Asp Asn Lys Val Val Cys Ser Cys Thr
               100                 105                 110
   Glu Gly Tyr Arg Leu Ala Glu Asn Gln Lys Ser Cys Glu Pro Ala Val
           115                 120                 125
   Pro Phe Pro Cys Gly Arg Val Ser Val Ser Gln Thr Ser Lys Leu Thr
       130                 135                 140
   Arg Ala Glu Thr Val Phe Pro Asp Val Asp Tyr Val Asn Ser Thr Glu
   145                 150                 155                 160
   Ala Glu Gly Ser Pro Gly Ser Gly Ser Ala Asn Ala Thr Gly Pro Ser
                   165                 170                 175
   Gly Glu Gly Ser Ala Pro Ser Glu Gly Asn Ala Thr Gly Pro Gly Thr
               180                 185                 190
   Ser Gly Gly Ser Pro Ala Asn Ser Thr Gly Gly Ser Pro Ala Glu Gly
           195                 200                 205
   Ser Pro Gly Ser Glu Ile Leu Asp Asn Ile Thr Gln Ser Thr Gln Ser
       210                 215                 220
   Phe Asn Asp Phe Thr Arg Val Val Gly Gly Glu Asp Ala Lys Pro Gly
   225                 230                 235                 240
   Gln Phe Pro Trp Gln Val Val Leu Asn Gly Lys Val Asp Ala Phe Cys
                   245                 250                 255
   Gly Gly Ser Ile Val Asn Glu Lys Trp Ile Val Thr Ala Ala His Cys
               260                 265                 270
   Val Glu Thr Gly Val Lys Ile Thr Val Val Ala Gly Glu His Asn Ile
           275                 280                 285
   Glu Glu Thr Glu His Thr Glu Gln Lys Arg Asn Val Ile Arg Ile Ile
       290                 295                 300
   Pro His His Asn Tyr Asn Ala Thr Ile Asn Lys Tyr Asn His Asp Ile
   305                 310                 315                 320
   Ala Leu Leu Glu Leu Asp Glu Pro Leu Val Leu Asn Ser Tyr Val Thr
                   325                 330                 335
   Pro Ile Cys Ile Ala Asp Lys Glu Tyr Thr Asn Ile Phe Leu Lys Phe
               340                 345                 350
   Gly Ser Gly Tyr Val Ser Gly Trp Gly Arg Val Phe His Lys Gly Arg
           355                 360                 365
   Ser Ala Leu Val Leu Gln Tyr Leu Arg Val Pro Leu Val Asp Arg Ala
       370                 375                 380
   Thr Cys Leu Xaa Ser Thr Lys Phe Thr Ile Tyr Asn Asn Met Phe Cys
   385                 390                 395                 400
   Ala Gly Phe His Glu Gly Gly Arg Asp Ser Cys Gln Gly Asp Ser Gly
                   405                 410                 415
   Gly Pro His Val Thr Glu Val Glu Gly Thr Ser Phe Leu Thr Gly Ile
               420                 425                 430
   Ile Ser Trp Gly Glu Glu Cys Ala Met Lys Gly Lys Tyr Gly Ile Tyr
           435                 440                 445
   Thr Lys Val Ser Arg Tyr Val Asn Trp Ile Lys Glu Lys Thr Lys Leu
       450                 455                 460
   Thr
   465
<210> 3
   <211> 415
   <212> PRT
   <213> Artificial
<220>
   <223> Modified Factor IX protein sequence
<220>
   <221> misc_feature
   <222> (338)..(338)
   <223> Xaa can be any naturally occurring amino acid
<400> 3
   Tyr Asn Ser Gly Arg Leu Glu Glu Phe Val Gln Gly Asn Leu Glu Arg
   1               5                   10                  15
   Glu Cys Met Glu Glu Lys Cys Ser Phe Glu Glu Ala Arg Glu Val Phe
               20                  25                  30
   Glu Asn Thr Glu Arg Thr Thr Glu Phe Trp Lys Gln Tyr Val Asp Gly
           35                  40                  45
   Asp Gln Cys Glu Ser Asn Pro Cys Leu Asn Gly Gly Ser Cys Lys Asp
       50                  55                  60
   Asp Ile Asn Ser Tyr Glu Cys Trp Cys Pro Phe Gly Phe Glu Gly Lys
   65                  70                  75                  80
   Asn Cys Glu Leu Asp Val Thr Cys Asn Ile Lys Asn Gly Arg Cys Glu
                   85                  90                  95
   Gln Phe Cys Lys Asn Ser Ala Asp Asn Lys Val Val Cys Ser Cys Thr
               100                 105                 110
   Glu Gly Tyr Arg Leu Ala Glu Asn Gln Lys Ser Cys Glu Pro Ala Val
           115                 120                 125
   Pro Phe Pro Cys Gly Arg Val Ser Val Ser Gln Thr Ser Lys Leu Thr
       130                 135                 140
   Arg Ala Glu Thr Val Phe Pro Asp Val Asp Tyr Val Asn Ser Thr Glu
   145                 150                 155                 160
   Ala Glu Thr Ile Leu Asp Asn Ile Thr Gln Ser Thr Gln Ser Phe Asn
                   165                 170                 175
   Asp Phe Thr Arg Val Val Gly Gly Glu Asp Ala Lys Pro Gly Gln Phe
               180                 185                 190
   Pro Trp Gln Val Val Leu Asn Gly Lys Val Asp Ala Phe Cys Gly Gly
           195                 200                 205
   Ser Ile Val Asn Glu Lys Trp Ile Val Thr Ala Ala His Cys Val Glu
       210                 215                 220
   Thr Gly Val Lys Ile Thr Val Val Ala Gly Glu His Asn Ile Glu Glu
   225                 230                 235                 240
   Thr Glu His Thr Glu Gln Lys Arg Asn Val Ile Arg Ile Ile Pro His
                   245                 250                 255
   His Asn Tyr Asn Ala Ala Ile Asn Lys Tyr Asn His Asp Ile Ala Leu
               260                 265                 270
   Leu Glu Leu Asp Glu Pro Leu Val Leu Asn Ser Tyr Val Thr Pro Ile
           275                 280                 285
   Cys Ile Ala Asp Lys Glu Tyr Thr Asn Ile Phe Leu Lys Phe Gly Ser
       290                 295                 300
   Gly Tyr Val Ser Gly Trp Gly Arg Val Phe His Lys Gly Arg Ser Ala
   305                 310                 315                 320
   Leu Val Leu Gln Tyr Leu Arg Val Pro Leu Val Asp Arg Ala Thr Cys
                   325                 330                 335
   Leu Xaa Ser Thr Lys Phe Thr Ile Tyr Asn Asn Met Phe Cys Ala Gly
               340                 345                 350
   Phe His Glu Gly Gly Arg Asp Ser Cys Gln Gly Asp Ser Gly Gly Pro
           355                 360                 365
   His Val Thr Glu Val Glu Gly Thr Ser Phe Leu Thr Gly Ile Ile Ser
       370                 375                 380
   Trp Gly Glu Glu Cys Ala Met Lys Gly Lys Tyr Gly Ile Tyr Thr Lys
   385 390 395 400
   Val Ser Arg Tyr Val Asn Trp Ile Lys Glu Lys Thr Lys Leu Thr
   405 410 415
<210> 4
   <211> 1536
   <212> DNA
   <213> Artificial
<220>
   <223> Factor IX 24-K5R codon optimized coding sequence with propeptide sequence
<400> 4
   atgcagcggg tgaatatgat catggctgag agtccaggac ttatcaccat atgcttgctg      60
   gggtatctcc tctccgctga gtgcaccgta ttcctcgatc acgagaacgc caacaaaatc     120
   cttaacagac gtaggcgata caacagtggc cgactggagg agtttgtcca aggtaacctg     180
   gaacgggaat gtatggagga gaagtgtagt ttcgaggagg ctcgggaggt gtttgagaac     240
   acagaaagaa caaccgaatt ttggaagcaa tatgtcgatg gtgaccaatg tgagtctaac     300
   ccttgtctta atggaggctc atgcaaagac gacattaaca gttatgaatg ttggtgtccc     360
   tttggcttcg agggaaagaa ttgtgagctg gacgtgacct gcaatattaa gaacggaagg     420
   tgcgagcagt tttgcaaaaa cagtgctgat aacaaggtgg tatgttcttg caccgaaggt     480
   taccgtcttg ctgaaaatca gaagagctgt gaaccagccg ttccctttcc ctgtggacgt     540
   gtaagcgttt ctcagacatc aaaactgacc cgggctgaga ctgtgttccc tgacgtcgat     600
   tacgttaact ctaccgaagc cgaaggaagc cccggcagcg ggtcagctaa cgcaaccggc     660
   cctagcggtg aaggctccgc tccttccgaa ggaaacgcaa ccggaccagg tacctccgga     720
   ggaagcccag ccaactccac aggggggtcc cctgccgagg ggagccctgg cagtgagatc     780
   ctggataaca tcacacagag cacacagagc tttaatgact tcacccgtgt ggtgggaggc     840
   gaggatgcaa agcccggaca gtttccatgg caggtggtcc tgaacggcaa ggtggatgcc     900
   ttttgcggag gatctatcgt gaatgaaaag tggattgtga ctgctgccca ctgtgtggag     960
   actggtgtga aaatcactgt ggtagcagga gaacacaata ttgaggagac cgagcatacc    1020
   gagcagaagc gcaatgtgat ccgcatcata cctcaccata actacaatgc aacaattaat    1080
   aagtacaacc atgacatcgc cctgttggag ctggatgagc ccctggtgct caattcttat    1140
   gtgacaccaa tctgcatagc tgacaaggaa tacactaaca ttttcctgaa gtttggcagt    1200
   ggatacgtgt caggatgggg cagagtgttc cacaagggac gctctgctct cgtgcttcag    1260
   tacctgcgag tgcctttggt ggatcgggca acatgtttga ggagcacaaa atttactatt    1320
   tacaacaata tgttttgcgc cggcttccac gaaggagggc gagattcatg ccagggagac    1380
   agtggcggtc cacacgtgac tgaagtcgaa ggcacctctt ttttgaccgg aatcatctct    1440
   tggggtgaag agtgtgccat gaaaggaaag tatggcatat acacaaaggt gtcccgctat    1500
   gtgaactgga tcaaggagaa gaccaaactc acctag                              1536
<210> 5
   <211> 1536
   <212> DNA
   <213> Artificial
<220>
   <223> Factor IX 24-K5R codon optimized coding sequence with propeptide sequence and any substitution at R338
<220>
   <221> misc_feature
   <222> (1300)..(1302)
   <223> n can be a, c, g, or t, so long as the codon comprising nucleotides 1300-1302 does not code for Arg
<400> 5
   atgcagcggg tgaatatgat catggctgag agtccaggac ttatcaccat atgcttgctg      60
   gggtatctcc tctccgctga gtgcaccgta ttcctcgatc acgagaacgc caacaaaatc     120
   cttaacagac gtaggcgata caacagtggc cgactggagg agtttgtcca aggtaacctg     180
   gaacgggaat gtatggagga gaagtgtagt ttcgaggagg ctcgggaggt gtttgagaac     240
   acagaaagaa caaccgaatt ttggaagcaa tatgtcgatg gtgaccaatg tgagtctaac     300
   ccttgtctta atggaggctc atgcaaagac gacattaaca gttatgaatg ttggtgtccc     360
   tttggcttcg agggaaagaa ttgtgagctg gacgtgacct gcaatattaa gaacggaagg     420
   tgcgagcagt tttgcaaaaa cagtgctgat aacaaggtgg tatgttcttg caccgaaggt     480
   taccgtcttg ctgaaaatca gaagagctgt gaaccagccg ttccctttcc ctgtggacgt     540
   gtaagcgttt ctcagacatc aaaactgacc cgggctgaga ctgtgttccc tgacgtcgat     600
   tacgttaact ctaccgaagc cgaaggaagc cccggcagcg ggtcagctaa cgcaaccggc     660
   cctagcggtg aaggctccgc tccttccgaa ggaaacgcaa ccggaccagg tacctccgga     720
   ggaagcccag ccaactccac aggggggtcc cctgccgagg ggagccctgg cagtgagatc     780
   ctggataaca tcacacagag cacacagagc tttaatgact tcacccgtgt ggtgggaggc     840
   gaggatgcaa agcccggaca gtttccatgg caggtggtcc tgaacggcaa ggtggatgcc     900
   ttttgcggag gatctatcgt gaatgaaaag tggattgtga ctgctgccca ctgtgtggag     960
   actggtgtga aaatcactgt ggtagcagga gaacacaata ttgaggagac cgagcatacc    1020
   gagcagaagc gcaatgtgat ccgcatcata cctcaccata actacaatgc aacaattaat    1080
   aagtacaacc atgacatcgc cctgttggag ctggatgagc ccctggtgct caattcttat    1140
   gtgacaccaa tctgcatagc tgacaaggaa tacactaaca ttttcctgaa gtttggcagt    1200
   ggatacgtgt caggatgggg cagagtgttc cacaagggac gctctgctct cgtgcttcag    1260
   tacctgcgag tgcctttggt ggatcgggca acatgtttgn nnagcacaaa atttactatt    1320
   tacaacaata tgttttgcgc cggcttccac gaaggagggc gagattcatg ccagggagac    1380
   agtggcggtc cacacgtgac tgaagtcgaa ggcacctctt ttttgaccgg aatcatctct    1440
   tggggtgaag agtgtgccat gaaaggaaag tatggcatat acacaaaggt gtcccgctat    1500
   gtgaactgga tcaaggagaa gaccaaactc acctag                               1536
<210> 6
   <211> 511
   <212> PRT
   <213> Artificial
<220>
   <223> Factor IX protein sequence with propeptide sequence
<400> 6
   Met Gln Arg Val Asn Met Ile Met Ala Glu Ser Pro Gly Leu Ile Thr
   1               5                   10                  15
   Ile Cys Leu Leu Gly Tyr Leu Leu Ser Ala Glu Cys Thr Val Phe Leu
               20                  25                  30
   Asp His Glu Asn Ala Asn Lys Ile Leu Asn Arg Arg Arg Arg Tyr Asn
           35                  40                  45
   Ser Gly Lys Leu Glu Glu Phe Val Gln Gly Asn Leu Glu Arg Glu Cys
       50                  55                  60
   Met Glu Glu Lys Cys Ser Phe Glu Glu Ala Arg Glu Val Phe Glu Asn
   65                  70                  75                  80
   Thr Glu Arg Thr Thr Glu Phe Trp Lys Gln Tyr Val Asp Gly Asp Gln
                   85                  90                  95
   Cys Glu Ser Asn Pro Cys Leu Asn Gly Gly Ser Cys Lys Asp Asp Ile
               100                 105                 110
   Asn Ser Tyr Glu Cys Trp Cys Pro Phe Gly Phe Glu Gly Lys Asn Cys
           115                 120                 125
   Glu Leu Asp Val Thr Cys Asn Ile Lys Asn Gly Arg Cys Glu Gln Phe
       130                 135                 140
   Cys Lys Asn Ser Ala Asp Asn Lys Val Val Cys Ser Cys Thr Glu Gly
   145                 150                 155                 160
   Tyr Arg Leu Ala Glu Asn Gln Lys Ser Cys Glu Pro Ala Val Pro Phe
                   165                 170                 175
   Pro Cys Gly Arg Val Ser Val Ser Gln Thr Ser Lys Leu Thr Arg Ala
               180                 185                 190
   Glu Thr Val Phe Pro Asp Val Asp Tyr Val Asn Ser Thr Glu Ala Glu
           195                 200                 205
   Gly Ser Pro Gly Ser Gly Ser Ala Asn Ala Thr Gly Pro Ser Gly Glu
       210                 215                 220
   Gly Ser Ala Pro Ser Glu Gly Asn Ala Thr Gly Pro Gly Thr Ser Gly
   225                 230                 235                 240
   Gly Ser Pro Ala Asn Ser Thr Gly Gly Ser Pro Ala Glu Gly Ser Pro
                   245                 250                 255
   Gly Ser Glu Ile Leu Asp Asn Ile Thr Gln Ser Thr Gln Ser Phe Asn
               260                 265                 270
   Asp Phe Thr Arg Val Val Gly Gly Glu Asp Ala Lys Pro Gly Gln Phe
           275                 280                 285
   Pro Trp Gln Val Val Leu Asn Gly Lys Val Asp Ala Phe Cys Gly Gly
       290                 295                 300
   Ser Ile Val Asn Glu Lys Trp Ile Val Thr Ala Ala His Cys Val Glu
   305                 310                 315                 320
   Thr Gly Val Lys Ile Thr Val Val Ala Gly Glu His Asn Ile Glu Glu
                   325                 330                 335
   Thr Glu His Thr Glu Gln Lys Arg Asn Val Ile Arg Ile Ile Pro His
               340                 345                 350
   His Asn Tyr Asn Ala Thr Ile Asn Lys Tyr Asn His Asp Ile Ala Leu
           355                 360                 365
   Leu Glu Leu Asp Glu Pro Leu Val Leu Asn Ser Tyr Val Thr Pro Ile
       370                 375                 380
   Cys Ile Ala Asp Lys Glu Tyr Thr Asn Ile Phe Leu Lys Phe Gly Ser
   385                 390                 395                 400
   Gly Tyr Val Ser Gly Trp Gly Arg Val Phe His Lys Gly Arg Ser Ala
                   405                 410                 415
   Leu Val Leu Gln Tyr Leu Arg Val Pro Leu Val Asp Arg Ala Thr Cys
   420 425 430
   Leu Arg Ser Thr Lys Phe Thr Ile Tyr Asn Asn Met Phe Cys Ala Gly
   435 440 445
   Phe His Glu Gly Gly Arg Asp Ser Cys Gln Gly Asp Ser Gly Gly Pro
   450 455 460
   His Val Thr Glu Val Glu Gly Thr Ser Phe Leu Thr Gly Ile Ile Ser
   465 470 475 480
   Trp Gly Glu Glu Cys Ala Met Lys Gly Lys Tyr Gly Ile Tyr Thr Lys
   485 490 495
   Val Ser Arg Tyr Val Asn Trp Ile Lys Glu Lys Thr Lys Leu Thr
   500 505 510
<210> 7
   <211> 511
   <212> PRT
   <213> Artificial
<220>
   <223> Factor IX protein sequence with propeptide sequence with K51R substitution and R434X substitution
<220>
   <221> misc_feature
   <222> (434)..(434)
   <223> Xaa can be any naturally occurring amino acid
<400> 7
   Met Gln Arg Val Asn Met Ile Met Ala Glu Ser Pro Gly Leu Ile Thr
   1 5 10 15
   Ile Cys Leu Leu Gly Tyr Leu Leu Ser Ala Glu Cys Thr Val Phe Leu
   20 25 30
   Asp His Glu Asn Ala Asn Lys Ile Leu Asn Arg Arg Arg Arg Tyr Asn
   35 40 45
   Ser Gly Arg Leu Glu Glu Phe Val Gln Gly Asn Leu Glu Arg Glu Cys
   50 55 60
   Met Glu Glu Lys Cys Ser Phe Glu Glu Ala Arg Glu Val Phe Glu Asn
   65 70 75 80
   Thr Glu Arg Thr Thr Glu Phe Trp Lys Gln Tyr Val Asp Gly Asp Gln
                   85                  90                  95
   Cys Glu Ser Asn Pro Cys Leu Asn Gly Gly Ser Cys Lys Asp Asp Ile
               100                 105                 110
   Asn Ser Tyr Glu Cys Trp Cys Pro Phe Gly Phe Glu Gly Lys Asn Cys
           115                 120                 125
   Glu Leu Asp Val Thr Cys Asn Ile Lys Asn Gly Arg Cys Glu Gln Phe
       130                 135                 140
   Cys Lys Asn Ser Ala Asp Asn Lys Val Val Cys Ser Cys Thr Glu Gly
   145                 150                 155                 160
   Tyr Arg Leu Ala Glu Asn Gln Lys Ser Cys Glu Pro Ala Val Pro Phe
                   165                 170                 175
   Pro Cys Gly Arg Val Ser Val Ser Gln Thr Ser Lys Leu Thr Arg Ala
               180                 185                 190
   Glu Thr Val Phe Pro Asp Val Asp Tyr Val Asn Ser Thr Glu Ala Glu
           195                 200                 205
   Gly Ser Pro Gly Ser Gly Ser Ala Asn Ala Thr Gly Pro Ser Gly Glu
       210                 215                 220
   Gly Ser Ala Pro Ser Glu Gly Asn Ala Thr Gly Pro Gly Thr Ser Gly
   225                 230                 235                 240
   Gly Ser Pro Ala Asn Ser Thr Gly Gly Ser Pro Ala Glu Gly Ser Pro
                   245                 250                 255
   Gly Ser Glu Ile Leu Asp Asn Ile Thr Gln Ser Thr Gln Ser Phe Asn
               260                 265                 270
   Asp Phe Thr Arg Val Val Gly Gly Glu Asp Ala Lys Pro Gly Gln Phe
           275                 280                 285
   Pro Trp Gln Val Val Leu Asn Gly Lys Val Asp Ala Phe Cys Gly Gly
       290                 295                 300
   Ser Ile Val Asn Glu Lys Trp Ile Val Thr Ala Ala His Cys Val Glu
   305                 310                 315                 320
   Thr Gly Val Lys Ile Thr Val Val Ala Gly Glu His Asn Ile Glu Glu
                   325                 330                 335
   Thr Glu His Thr Glu Gln Lys Arg Asn Val Ile Arg Ile Ile Pro His
               340                 345                 350
   His Asn Tyr Asn Ala Thr Ile Asn Lys Tyr Asn His Asp Ile Ala Leu
           355                 360                 365
   Leu Glu Leu Asp Glu Pro Leu Val Leu Asn Ser Tyr Val Thr Pro Ile
       370                 375                 380
   Cys Ile Ala Asp Lys Glu Tyr Thr Asn Ile Phe Leu Lys Phe Gly Ser
   385                 390                 395                 400
   Gly Tyr Val Ser Gly Trp Gly Arg Val Phe His Lys Gly Arg Ser Ala
                   405                 410                 415
   Leu Val Leu Gln Tyr Leu Arg Val Pro Leu Val Asp Arg Ala Thr Cys
               420                 425                 430
   Leu Xaa Ser Thr Lys Phe Thr Ile Tyr Asn Asn Met Phe Cys Ala Gly
           435                 440                 445
   Phe His Glu Gly Gly Arg Asp Ser Cys Gln Gly Asp Ser Gly Gly Pro
       450                 455                 460
   His Val Thr Glu Val Glu Gly Thr Ser Phe Leu Thr Gly Ile Ile Ser
   465                 470                 475                 480
   Trp Gly Glu Glu Cys Ala Met Lys Gly Lys Tyr Gly Ile Tyr Thr Lys
                   485                 490                 495
   Val Ser Arg Tyr Val Asn Trp Ile Lys Glu Lys Thr Lys Leu Thr
               500                 505                 510
<210> 8
   <211> 9
   <212> PRT
   <213> Artificial
<220>
   <223> Glycosylation site sequence
<400> 8
   Val Phe Ile Gln Asp Asn Ile Thr Asp
   1               5
<210> 9
   <211> 9
   <212> PRT
   <213> Artificial
<220>
   <223> Glycosylation site sequence
<400> 9
   Val Phe Ile Gln Asp Asn Ile Thr Asn
   1               5
<210> 10
   <211> 45
   <212> PRT
   <213> Artificial
<220>
   <223> Glycosylation site sequence
<400> 10
   Ala Glu Thr Val Phe Pro Asp Val Asp Tyr Val Asn Ser Thr Glu Asn
   1               5                   10                  15
   Glu Thr Ile Gln Asp Asn Ile Thr Asp Asn Glu Thr Ile Leu Asp Asn
               20                  25                  30
   Ile Thr Gln Ser Thr Gln Ser Phe Asn Asp Phe Thr Arg
           35                  40                  45
<210> 11
   <211> 35
   <212> PRT
   <213> Artificial
<220>
   <223> FIX activation peptide
<400> 11
   Ala Glu Thr Val Phe Pro Asp Val Asp Tyr Val Asn Ser Thr Glu Ala
   1               5                   10                  15
   Glu Thr Ile Leu Asp Asn Ile Thr Gln Ser Thr Gln Ser Phe Asn Asp
               20                  25                  30
   Phe Thr Arg
           35